# EUROPEAN PATENT SPECIFICATION

(11) **EP 3 829 663 B1**
(45) Date of publication and mention of the grant of the patent: **08.07.2026**
(21) Application number: 19845186.6
(22) Date of filing: 29.07.2019
(51) Int. Cl.: A61L 27/56, A61L 27/14, A61F 2/30, A61B 17/56

(54) **IMPLANTABLE THREE DIMENSIONAL TISSUE SCAFFOLDS**
IMPLANTIERBARE DREIDIMENSIONALE GEWEBEGERÜSTE
ÉCHAFAUDAGES TISSULAIRES TRIDIMENSIONNELS IMPLANTABLES

(30) Priority: 30.07.2018 US 201862711667 P; 04.12.2018 US 201862775228 P
(43) Date of publication of application: 09.06.2021
(73) Proprietor: Nanochon, Inc., Burke, VA 22015 (US)
(72) Inventor: HOLMES, Benjamin, Burke, VA 22015 (US); CASTRO, Nathan, Burke, VA 22015 (US)
(74) Representative: Brand Murray Fuller LLP
(86) International application number: PCT/US2019/043955
(87) International publication number: WO 2020/028268

(56) References cited:
- WO-A1-2010/044758
- WO-A1-2014/074134
- WO-A1-2014/204634
- US-A1- 2005 112 397
- US-A1- 2012 227 899
- US-A1- 2014 243 995
- US-A1- 2018 021 138
- US-A1- 2018 055 643

## Description

### BACKGROUND

Large, critical-sized tissue defects (e.g., those that are too large to heal naturally) caused by traumatic injury, cancer, or disease of tissue may be challenging to treat. These defects may often be associated with a low rate of recovery and high patient morbidity. Developing scaffolds may address this need.

WO 2014/074134 ("WO'134") discloses multilayered collagen scaffolds. In particular, WO'134 discloses a collagen scaffold comprising 2-300 stacked collagen fiber layers, wherein at least 90% of the collagen fibers in each layer have a length of at least 2 mm and are so oriented that they extend along the layer and are parallel to each other, each layer has a thickness of 1-100 µm and features a direction identical to the orientation of the at least 90% of the collagen fibers, and the angle between the directions of every collagen fiber layers next to each other is 30°-90°.

### BRIEF SUMMARY

The following presents a simplified summary of various aspects described herein. This summary is not an extensive overview and is not intended to identify key or critical elements or to delineate the scope of the claims. The following summary merely presents some concepts in a simplified form as an introductory prelude to the more detailed description provided below.

Provided herein are scaffolds that may be 3D printed, surgically implantable elastomeric microstructured scaffolds, nanostructured scaffolds or a combination thereof for promoting bone regeneration, vascular regeneration, cartilage regeneration, or a combination thereof at tissue regions, such as osteochondral regions.

In an aspect, the present invention provides a three-dimensional tissue scaffold comprising: (a) a first region comprising a plurality of layers, wherein at least a first layer of the plurality of layers is of a first rotational offset from at least a second layer of the plurality of layers; and (b) a second region comprising a plurality of layers, wherein at least a first layer of the plurality of layers is of a second rotational offset from at least a second layer of the plurality of layers; wherein the first rotational offset is greater than the second rotational offset; and (c) a third region, positioned in between the first region and the second region, wherein the third region comprises a plurality of layers, and wherein at least a first layer of the plurality of layers of the third region is of a first sinusoidal pattern at a first rotation, at least a second layer of the plurality of layers of the third region is of the first sinusoidal pattern at the second rotation, and at least a third layer of the plurality of layers of the third region is of the first sinusoidal pattern at the third rotation.

Alternative embodiments are set out in the dependent claims herein.

In some cases, the plurality of layers of the first region may further comprise a bottommost layer formed from at least a first boundary segment and at least a first crossing segment, and the plurality of layers of the second region may further comprise a topmost layer formed from at least a second boundary segment and a second crossing segment, and wherein an exterior surface of at least the first boundary segment and at least the first crossing segment of the bottommost layer may have a first topography comprising a plurality of peaks and valleys of a first average amplitude and an exterior surface of at least the second boundary segment and at least the second crossing segment of the topmost layer may have a second topography comprising a plurality of peaks and valleys of a second average amplitude.

In some cases, the first average amplitude may be greater than the second average amplitude. In some cases, at least the first layer of the plurality of layers of the first region may be of a first sinusoidal pattern at a first rotation and at least the second layer of the plurality of layers of the first region may be of the first sinusoidal pattern at a second rotation. In some cases, at least the first layer and second layer of the plurality of layers of the first region may be formed from a first number of boundary segments and a first number of crossing segments. In some cases, at least the first layer of the plurality of layers of the second region may be of a second sinusoidal pattern at the first rotation and at least the second layer of the plurality of layers of the second region may be of the second sinusoidal pattern at a third rotation. In some cases, at least the first layer and second layer of the plurality of layers of the second region may be formed from a second number of boundary segments and a second number of crossing segments. In some cases, the first number of boundary segments may be less than the second number of boundary segments and the first number of crossing segments may be less than the second number of boundary segments.

In some cases, each of the plurality of layers of the third region may comprise a number of boundary segments and a number of crossing segments, and wherein the number of boundary segments and the number of crossing segments may increase on a layer-by-layer basis from a first number of boundary segments and a first number of crossing segments of the first layer of the plurality of layers of the third region to a second number of boundary segments and a second number of crossing segments of the last layer of the plurality of layers of the third region. In some cases, the first number of boundary segments and the first number of crossing segments of the first layer of the plurality of layers of the third region may be equal to the first number of boundary segments and the first number of crossing segments of the first layer of the plurality of layers of the first region. In some cases, the second number of boundary segments and the second number of crossing segments of the last layer of the plurality of layers of the third region may be equal to the second number of boundary segments and the second number of crossing segments of the first layer of the plurality of layers of the second region.

Disclosed herein, but not claimed, is a method of manufacturing a three-dimensional scaffold. In some cases, the method may comprise (i) fabricating a first region by printing a plurality of layers, wherein at least a first layer of the plurality of layers may be of a first rotational offset from at least a second layer of the plurality of layers; and (ii) fabricating a second region by printing a plurality of layers, wherein at least a first layer of the plurality of layers may be of a second rotational offset from at least a second layer of the plurality of layers; wherein the first rotational offset may be greater than the second rotational offset. In some cases, the plurality of layers of the first region further may comprise a bottommost layer formed from at least a first boundary segment and at least a first crossing segment, and the plurality of layers of the second region further may comprise a topmost layer formed from at least a second boundary segment and a second crossing segment, and wherein an exterior surface of at least the first boundary segment and at least the first crossing segment of the bottommost layer may have a first topography comprising a plurality of peaks and valleys of a first average amplitude and an exterior surface of at least the second boundary segment and at least the second crossing segment of the topmost layer may have a second topography comprising a plurality of peaks and valleys of a second average amplitude. In some cases, the first average amplitude may be greater than the second average amplitude. In some cases, at least the first layer and second layer of the plurality of layers of the first region may be formed from a first number of boundary segments and a first number of crossing segments. In some cases, at least the first layer and second layer of the plurality of second layers may be formed from a second number of boundary segments and a second number of crossing segments. In some cases, the first number of boundary segments may be less than the second number of boundary segments and the first number of crossing segments may be less than the second number of boundary segments. In some cases, the three-dimensional scaffold may be printed from a first material having at least a soluble component and an insoluble component, and the soluble component of the first material may be soluble in water.

Disclosed herein, but not claimed, is a method of treating a subject having a tissue defect. In some cases, the method comprising: (i) surgically implanting a three-dimensional tissue scaffold into the tissue defect of the subject, thereby treating the subject, wherein the three-dimensional tissue scaffold may comprise: (a) a first region comprising a plurality of layers, wherein at least a first layer of the plurality of layers may be of a first rotational offset from at least a second layer of the plurality of layers; and (b) a second region comprising a plurality of layers, wherein at least a first layer of the plurality of layers may be of a second rotational offset from at least a second layer of the plurality of layers; wherein the first rotational offset may be greater than the second rotational offset.

A first layer of the plurality of first layers may be of a first sinusoidal pattern at a first rotation and a second layer of the plurality of first layers may be of the first sinusoidal pattern at a second rotation.

In some embodiments, the first layer and the second layer of the first plurality of layers may be formed from a first number of boundary segments and a first number of crossing segments.

A first layer of the plurality of second layers may be of a second sinusoidal pattern at the first rotation and a second layer of the plurality of second layers may be of the second sinusoidal pattern at the second rotation.

In some embodiments, the first layer and the second layer of the second plurality of layers may be formed from a second number of boundary segments and a second number of crossing segments.

In some embodiments, the first number of boundary segments may be less than the second number of boundary segments and the first number of crossing segments may be less than the second number of boundary segments.

In some embodiments, each of the plurality of layers of the third region comprises a number of boundary segments and a number of crossing segments, and wherein the number of boundary segments and the number of crossing segments may increase on a layer-by-layer basis from a first number of boundary segments and a first number of crossing segments of the first layer of the plurality of layers of the third region to a second number of boundary segments and a second number of crossing segment of the last layer of the plurality of layers of the third region.

In some embodiments, the first number of boundary segments and the first number of crossing segments of the first layer of the plurality of layers of the third region is equal to the first number of boundary segments and the first number of crossing segments of the first layer of the plurality of layers of the first region..

In some embodiments, the second number of boundary segments and the second number of crossing segments of the last layer of the plurality of layers of the third region is equal to the second number of boundary segments and the second number of crossing segments of the first layer of the plurality of layers of the second region..

In some embodiments, the first tissue may be bone, and the second tissue may be cartilage.

Disclosed herein, but not claimed, is a method of manufacturing a three-dimensional scaffold may comprise fabricating a first region by printing a first plurality of layers, wherein at least a first layer of the plurality of first layers is of a first rotational offset from at least a second layer of the plurality of first layers, and fabricating a second region by printing a second plurality of layers, wherein at least a first layer of the plurality of second layers is of a second rotational offset from at least a second layer of the plurality of second layers, wherein the first rotational offset may be greater than the second rotational offset.

In some embodiments, the method may further comprise fabricating a third region by printing a third plurality of layers, wherein each of the third plurality of layers has a third number of boundary segments and third number of crossing segments.

In some embodiments, the first region may be configured to configured to promote tissue growth of a first tissue, the third region may be configured to configured to promote tissue growth of a second tissue, and the second region may be configured to promote tissue growth of along a transitionary region between the first tissue and the second tissue.

In some embodiments, the first number of boundary segments and the first number of crossing segments may be less than the third number of boundary segments and the third number of crossing segments.

In some embodiments, the three-dimensional scaffold may be printed from a first material having at least a soluble component and an insoluble component and the soluble component of the first material may be soluble in water.

Disclosed herein, but not claimed, is a method of treating a subject having a tissue defect is provided and the method may comprise surgically implanting a three-dimensional tissue scaffold into the tissue defect of the subject, thereby treating the subject, wherein the three-dimensional tissue scaffold may comprise a first region including a plurality of first layers, wherein at least a first layer of the plurality of first layers may be of a first rotational offset from at least a second layer of the plurality of first layers, a second region including a plurality of second layers, wherein at least a first layer of the plurality of second layers may be of a second rotational offset from at least a second layer of the plurality of second layers, wherein the first rotational offset may be greater than the second rotational offset.

In some cases, the scaffold may comprise a first region configured to promote tissue growth of a first tissue; a second region configured to promote tissue growth of a second tissue; and a third region, positioned between the first region and the second region, configured to promote tissue growth at a transitionary region between the first tissue and the second tissue. A first layer of the plurality of first layers may be of a first sinusoidal pattern at a first rotation and a second layer of the plurality of first layers may be of the first sinusoidal pattern at a second rotation. In some cases, the first layer and the second layer of the first plurality of layers may be formed from a first number of boundary segments and a first number of crossing segments. A first layer of the plurality of second layers may be of a second sinusoidal pattern at the first rotation and a second layer of the plurality of second layers may be of the second sinusoidal pattern at the second rotation. In some cases, the first layer and the second layer of the second plurality of layers may be formed from a second number of boundary segments and a second number of crossing segments. In some cases, the first number of boundary segments may be less than the second number of boundary segments and the first number of crossing segments may be less than the second number of boundary segments In some cases, a number of boundary segments and a number of crossing segments may increase on a layer-by-layer basis from the first layer of the plurality of third layers to a last layer of the plurality of third layers. In some cases, a number of boundary segments and a number of crossing segments of the first layer of the third plurality of layers may be equal to the first number of boundary segments and the first number of crossing segments of the first layer. In some cases, a number of boundary segments and a number of crossing segments of the last layer of the third plurality of layers may be equal to the second number of boundary segments and the second number of crossing segments of the first layer of the second plurality of layers of the second region. In some cases, the first tissue may be bone. In some cases, the second tissue may be cartilage.

Disclosed herein, but not claimed, is a method of manufacturing a three-dimensional scaffold. In some cases, the method may comprise fabricating a first region by printing a first plurality of layers, wherein each of the first plurality of layers has a first number of boundary segments and a first number of crossing segments; and fabricating a second region by printing a second plurality of layers, wherein each of the second plurality of layers has a second number of boundary segments and a second number of crossing segments. In some cases, the method may further comprise fabricating a third region by printing a third plurality of layers, wherein each of the third plurality of layers may have a third number of boundary segments and third number of crossing segments. In some cases, the first region may be configured to configured to promote tissue growth of a first tissue, the third region may be configured to configured to promote tissue growth of a second tissue, and the second region may be configured to promote tissue growth of along a transitionary region between the first tissue and the second tissue. In some cases, the second region may be positioned between the first region and the third region. In some cases, the first number of boundary segments and the first number of crossing segments may be less than the third number of boundary segments and the third number of crossing segments. In some cases, the three-dimensional scaffold may be printed from a first material having at least a soluble component and an insoluble component. In some cases, the soluble component of the first material may be soluble in water.

Disclosed herein, but not claimed, is a method of treating a subject having a tissue defect. In some cases, the method may comprise surgically implanting a three-dimensional tissue scaffold into the tissue defect of the subject, thereby treating the subject, wherein the three-dimensional tissue scaffold comprises: a first region configured to promote tissue growth of a first tissue; a second region configured to promote tissue growth of a second tissue; and a third region, positioned between the first region and the second region, configured to promote tissue growth at a transitionary region between the first tissue and the second tissue.

### BRIEF DESCRIPTION OF THE DRAWINGS

A more complete understanding of aspects described herein and the advantages thereof may be acquired by referring to the following description in consideration of the accompanying drawings, in which like reference numbers indicate like features.
Figure 1 depicts a bottom perspective view of an exemplary tissue scaffold according to one or more embodiments of the present disclosure.
Figure 2A depicts a plurality of individual layers of an exemplary tissue scaffold according to one or more embodiments of the present disclosure.
Figure 2B depicts a plurality of regions of an exemplary tissue scaffold, wherein each of the plurality of regions are comprised of one or more of the plurality of layers depicted in Figure 2A, according to one or more embodiments of the present disclosure.
Figures 3A, 3B, 3C, and 3D respectively depict a bottom view, a top view, a bottom perspective view, and a top perspective view of a first exemplary tissue scaffold according to one or more embodiments of the present disclosure.
Figures 4A, 4B, 4C, and 4D respectively depict a bottom view, a top view, a bottom perspective view, and a top perspective view of a second exemplary tissue scaffold according to one or more embodiments of the present disclosure.
Figures 5A and 5B respectively depict exemplary nano- and/or micro- scale topographical views of a topmost layer and a bottommost layer of an exemplary tissue scaffold according to one or more embodiments of the present disclosure.
Figure 6 depicts a non-limiting example of a tissue scaffold that may be implanted arthroscopically into an osteochondral defect according to one or more embodiments of the present disclosure.
Figure 7 depicts a non-limiting flow diagram illustrating an exemplary method of fabricating a tissue scaffold as disclosed herein but not claimed.
Figure 8 depicts a non-limiting example of a procedure for implanting a tissue engineering scaffold arthroscopically into an osteochondral defect as disclosed herein but not claimed.
Figure 9 depicts a graph of the Young's modulus of exemplary solid and porous tissue engineering scaffolds under wetted, not wetted, and wetted and dried conditions.
Figure 10 depicts graphs of the fatigue profile of an exemplary wetted tissue scaffold during cycle loading.
Figures 11A, 11B, 11C, and 11D depict scanning electron microscopy (e.g., SEM) images of an exemplary wetted tissue scaffold after cycle loading.
Figure 12 depicts a plurality of exemplary femoral condyle cross-sections with microfracture, solid thermoplastic polyurethane (e.g., nTPU) implant, and 3D printed nanoporous nTPU implant treatments.
Figure 13A depicts an exemplary femoral condyle cross-section with microfracture treatment after a period of time.
Figure 13B depicts a hematoxylin and eosin (e.g., H&E) histological stain of the microfracture treatment area after the period of time.
Figure 14A depicts an exemplary femoral condyle cross-section with solid nTPU implant treatment after a period of time.
Figure 14B depicts an H&E histological stain of the solid nTPU implant treatment area after the period of time.
Figure 15A depicts an exemplary femoral condyle cross-section with 3D printed nanoporous nTPU implant treatment after a period of time.
Figure 15B depicts an H&E histological stain of the 3D printed nanoporous nTPU implant treatment area after the period of time.
Figure 16 depicts a graph of the Young's modulus of healthy tissue, and microfracture, solid nTPU implant, and 3D printed nanoporous nTPU implant treatment tissue areas.
Figures 17A, 17B, 17C, and 17D depict H&E histological stains of chondrocyte clusters and new tissue at the 3D printed nanoporous nTPU implant treatment area after the period of time.

### DETAILED DESCRIPTION

In the following description of the various examples and components of this disclosure, reference is made to the accompanying drawings, which form a part hereof, and in which are shown by way of illustration various example structures and environments in which aspects of the disclosure may be practiced. It is to be understood that other structures and environments may be utilized and that structural and functional modifications may be made from the specifically described structures and methods.

### Customizing Layers of a Scaffold for Multi-Tissue-Type Integration

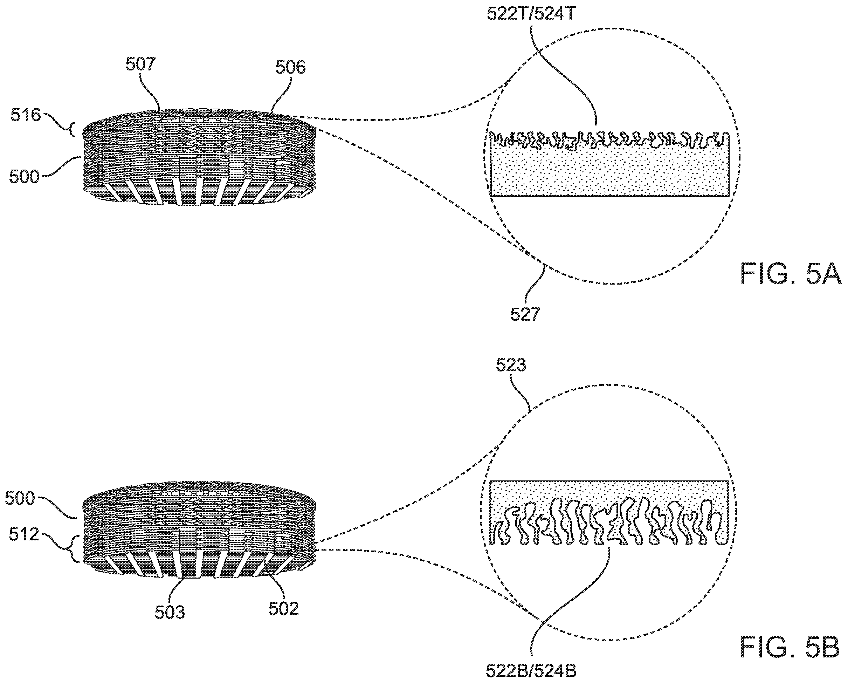

As provided herein, a scaffold may be comprised by a plurality of regions. A region of the plurality of regions may comprise one or more layers, such as a plurality of layers. In some cases, each region of the plurality of regions may comprise a plurality of layers. In forming the plurality of regions of the scaffold, layers may be deposited on a layer-by-layer basis. A layer of a plurality of layers may comprise one or more parameters such as a material composition, a deposited volume, a geometric pattern, a degree of rotation relative to a common frame of reference, or any combination thereof. In some cases, a layer may comprise a parameter that is different from another layer. For example, a first layer may comprise a geometric pattern that may be different from a geometric pattern of a second layer. In some cases, a layer may comprise a parameter that is the same as another layer. For example, a first layer may comprise a deposited volume that may be the same as a deposited volume of a second layer. One or more parameters of a layer may be the same as another layer, one or more parameters of a layer may be different from another layer, or a combination thereof. A geometric feature of a second layer may be rotated relative to a common frame of reference in comparison of a geometric feature of a first layer. A region of layers may comprise one or more parameters suitable for a tissue region and a second region of layers may comprise one or more parameters suitable for a different tissue region. An end layer, such as a bottommost layer of a scaffold (such as deposited on a build try of an FDM rapid prototyping device) may have a smaller amplitude between a peak and a valley of a topographical surface of the material deposited in relation to an opposing end layer (such as a topmost layer) of the scaffold, which may be exposed or untouched by a contacting surface, such as shown in FIG. 5. When implanted, a layer having a smaller amplitude between one or more peaks and valleys may be configured to interface with an adjacent native tissue (such as a cartilage tissue) to advantageously provide an optimal surface for articulation (such as a substantially smooth surface). A scaffold may be configured with parameters such that upon implantation, a first region advantageously recruits a first native tissue to infiltrate and a second region advantageously recruits a second native tissue type to infiltrate.

### Producing Scaffolds with Layers of Varying Properties

Provided herein, but not claimed, are methods of making a scaffold, such as a 2D or 3D scaffold. A scaffold may be formed layer by layer. A layer may be deposited onto a second layer. A first layer and a second layer may be formed separately and joined together, such as by an adhesive. A composition of a first layer may differ from a composition of a second layer. For example, a scaffold may be produced having a ratio of polymer A and polymer B. At one end of the scaffold, a ratio of polymer A to polymer B may be higher that a ratio of polymer A to polymer B at an opposing end. In a different example, a ratio of polymer A to polymer B may be higher in a center region of the scaffold and lower at edge regions of the scaffold. Printing methods may be suitable for producing scaffolds as described herein, such as layer by layer printing. Rapid prototyping technologies may be suitable for producing scaffolds as described herein. One or more parameters of printing or rapid prototyping technologies may be adjusted before or during production of scaffolds as described herein.

### Deformable Scaffolds for Minimally Invasive Delivery

Provided herein, but not claimed, are scaffolds provided for implantation to a subject, such as a subject in need thereof. A subject may have a tissue defect. A scaffold may be configured for a tissue defect, such as substantially matching a volume or shape of the tissue defect. A scaffold may also be configured to be fitted into a delivery device for delivery to the site of the tissue defect. A delivery device may comprise a minimally invasive tool. Advantageously, a scaffold may be configured with elastic properties, shape memory properties, or a combination thereof, such that a scaffold may be manipulated or deformed to fit within a minimally invasive tool for delivery to a tissue defect, and upon delivery, reform, unbend, or return to an original shape or volume that substantially matches that of the tissue defect. A scaffold may exhibit flexible or elastic mechanical properties. A scaffold may be treated by one or more solvents for a period of time to modify or enhance a flexible or elastic mechanical property. A scaffold may be delivered via a non-invasive surgical technique, such as an arthroscopic tool. This may be an advantage over other scaffolds that may be delivered by an invasive surgical technique to the tissue defect, thereby creating a secondary injury site.

The term "about," as used herein, may refer to a range that is 15% greater than or less than a stated numerical value within the context of the particular usage. For example, "about 10" may include a range from 8.5 to 11.5.

The term "region," as used herein, may be interchangeable with any of a "composition", "construct", "component," "section," "area," "portion," or the like of a tissue scaffold. A tissue scaffold may be comprised of one or more regions, each of which having similar and/or unique structural and/or mechanical characteristics. The term "layer," as used herein, may be interchangeable with any of a "plane," "deposition," "increment," or the like and may refer to a single three-dimensional print effort by a rapid prototyping device as defined by material deposit at a particular vertical (e.g., z-axis) step and/or increment. One or more layers may comprise a region of a tissue scaffold. The term "tissue scaffold," as used herein, may be interchangeable with a "tissue engineering scaffold" and may refer to product produced by a rapid prototyping device for facilitating tissue regeneration. The term "insoluble component" as used herein may refer to a material that does not dissolve or does not substantially dissolve in a given solvent. The term "soluble component" as used herein may refer to a material that is capable of dissolving in a given solvent. The term "smoother," as used herein, may refer to a distance between peaks and valleys of a topographical surface having a smaller amplitude than a "rougher" surface having a greater amplitude between similar peaks and valleys.

A scaffold as described herein, but not claimed, may be substantially a two dimensional scaffold. A scaffold may be at least partially porous. A porosity may vary across a scaffold. A scaffold may be acellular. A scaffold may be at least partially cellularized. A scaffold may be configured to promote cellular infiltration upon implantation in a subject in need thereof. A scaffold may be configured for implantation at a site of a tissue defect, such as comprising a macroscopic shape or volume that substantially mirrors a shape or volume of the tissue defect. A scaffold may be configured to comprise an original shape and upon a force applied be deformable and upon removing the applied force the scaffold may be configured to return to the original shape. This feature may be advantageous for deformable a scaffold to fit into a surgical delivery tool (such as a minimally invasive surgical tool) for delivery to a tissue defect site and upon placement at the tissue defect site return to the original shape. This feature of deforming and returning to an original shape may be configured at least in part by one or more materials that are comprised by the scaffold. This feature may be configured at least in part by a geometric deposition pattern of the scaffold, a degree of rotation relative to a common frame of reference, a change in amplitude of a topographical surface of the scaffold, or any combination thereof. This feature may be configured at least in part by a rotational offset of one or more layers of the scaffold.

A scaffold may be configured for a tissue defect at a tissue interface. For example, a first portion of a scaffold may comprise features beneficial for a first tissue of the tissue interface and a second portion of the scaffold may comprise features beneficial for a second tissue of the tissue interface. The first and second portions may be positioned adjacent to one another. The first and second portions may be discrete portions. The first and second portions may form a gradient across a length of the scaffold. For example, a gradient of increasing porosity across the length. For example, a gradient of amplitude from smaller to larger amplitudes of peaks and valleys of a topographical surface of layers of a scaffold. A scaffold may comprise a gradient of porosity across a length of the scaffold.

A tissue defect as described herein may include any tissue having a non-native structure. A tissue defect may result from an injury sustained to an individual. A tissue defect may result from an infection or disease acquired by an individual. A tissue defect may result from a surgical resection or alteration of a tissue in an individual. A tissue defect may result from a natural aging process.

A tissue defect may span across one or more tissues. A tissue defect may span across an interface such as a bone to cartilage interface or muscle to cartilage interface. A tissue defect may span across an interface such as a vascularized tissue to an avascular tissue. A tissue defect may comprise an osteochondral defect.

Described herein are scaffolds for tissue regeneration, such as printed, surgically implantable scaffolds for facilitating tissue regeneration. The scaffolds may be three dimensional (3D). The scaffolds may be 3D printed. The scaffolds may be surgically implantable elastomeric microstructured or nanostructured tissue engineering scaffolds. The scaffolds may be configured for promoting bone, vascular, or cartilage regeneration at osteochondral regions or any combination thereof.

### Arthroscopically Implantable Tissue Scaffolds

An arthroscopically implantable three-dimensional tissue scaffold is provided.

In some embodiments, the three-dimensional tissue scaffolds disclosed herein may comprise at least a composite material that includes at least an insoluble component and a soluble component. The tissue scaffold may be exposed to, submerged under, and/or contacted with a solvent that may be capable of dissolving the soluble component, leaving the insoluble component intact and in substantially the same shape and/or size as fabricated. Non-limiting examples of solvents may include, without limitation, water (H₂O), acetic acid, acetone, acetonitrile, benzene, 1-butanol, 2-butanol, 2-butanone, t-butyl alcohol, carbon tetrachloride, chlorobenzene, chloroform, cyclohexane, 1,2-dichloroethane, diethylene glycol, diethyl ether, diglyme (diethylene glycol dimethyl ether), 1,2-dimethoxy-ethane (glyme, DME), dimethyl-formamide (DMF), dimethyl sulfoxide (DMSO), 1,4-dioxane, ethanol, ethyl acetate, ethylene glycol, glycerin, heptane, hexamethylphosphoramide (HMPA), hexamethylphosphorous triamide (HMPT), hexane, methanol, methyl t-butyl ether (MTBE), methylene chloride, N-methyl-2-pyrrolidinone (NMP), nitromethane, pentane, petroleum ether (ligroin), 1-propanol, 2-propanol, pyridine, tetrahydrofuran (THF), toluene, triethyl amine, o-xylene, m-xylene, p-xylene, and D-limonene.

In some cases, the three-dimensional tissue scaffold comprises a composite material having a component that is insoluble in water, and another component that is soluble in water. In this example, immersing the tissue scaffold in water may dissolve the soluble component, leaving the insoluble component intact. Non-limiting examples of water-insoluble components that may be used in the three-dimensional tissue scaffolds of the disclosure may include: thermoplastic polyurethane (TPU), polycaprolactone (PCL), poly co-glycolic acid (PLGA), polylactic acid (PLA), and high impact polystyrene (HIPS). Non-limiting examples of water-soluble components that may be used in the three-dimensional tissue scaffolds of the disclosure may include: polyvinyl alcohol (PVA), salt, sugar or sugar glass, polyethylene glycol (PEG) and any un-crosslinked functionalized derivative thereof (such as PEGDA), gelatin and any derivative thereof (such as gelatin methacrylate), poly co-glycolic acid (PLGA), alginate, and sodium bicarbonate and other effervescent materials. In some cases, the insoluble component may be thermoplastic polyurethane (TPU) and the soluble component may be polyvinyl alcohol (PVA).

In additional cases, the three-dimensional tissue scaffold the composite material described above may further include a component comprising hydrolyzable ester groups allowing for degradability of the insoluble material, as well as non-degradable ethers.

In order to accelerate the dissolving of the soluble component from the soluble component, the tissue scaffold may be shaken, agitated, moved, vibrated, and/or otherwise mechanically displaced within the solvent. Additionally and/or alternatively, a tissue scaffold composed of the material with the soluble and insoluble components may be fastened, attached, and/or otherwise fixed and the solvent may be shaken, agitated, moved, vibrated, and/or otherwise mechanically displaced around, over, and/or through the tissue scaffold.

The composite material may comprise an insoluble component in an amount ranging from about 50 wt % of the composite material to about 95 wt % of the composite material. For example, the insoluble component may be present in the composite material in the amount that is about: 50-95 wt %, 55-90 wt %, 60-85 wt %, 65-80 wt %, 60-80 wt %, 65-75 wt %, 50 wt %, 51 wt %, 52 wt %, 53 wt %, 54 wt %, 55 wt %, 56 wt %, 57 wt %, 58 wt %, 59 wt %, 60 wt %, 61 wt %, 62 wt %, 63 wt %, 64 wt %, 65 wt %, 66 wt %, 67 wt %, 68 wt %, 69 wt %, 70 wt %, 71 wt %, 72 wt %, 73 wt %, 74 wt %, 75 wt %, 76 wt %, 77 wt %, 78 wt %, 79 wt %, 80 wt %, 81 wt %, 82 wt %, 83 wt %, 84 wt %, 85 wt %, 86 wt %, 87 wt %, 88 wt %, 89 wt %, 90 wt %, 91 wt %, 92 wt %, 93 wt %, 94 wt %, or 95 wt % of the composite material. In some instances, however, the insoluble component of the composite material may range from about 5 wt % of the composite material to about 50 wt % of the composite material.

In some cases, the composite material may comprise the insoluble component in an amount that is at least about 50 wt % of the composite material. For example, the insoluble component may be present in the composite material in the amount that is at least about: 50 wt %, 51 wt %, 52 wt %, 53 wt %, 54 wt %, 55 wt %, 56 wt %, 57 wt %, 58 wt %, 59 wt %, 60 wt %, 61 wt %, 62 wt %, 63 wt %, 64 wt %, 65 wt %, 66 wt %, 67 wt %, 68 wt %, 69 wt %, 70 wt %, 71 wt %, 72 wt %, 73 wt %, 74 wt %, 75 wt %, 76 wt %, 77 wt %, 78 wt %, 79 wt %, 80 wt %, 81 wt %, 82 wt %, 83 wt %, 84 wt %, 85 wt %, 86 wt %, 87 wt %, 88 wt %, 89 wt %, 90 wt %, 91 wt %, 92 wt %, 93 wt %, 94 wt %, or 95 wt % of the composite material.

The composite material may comprise a soluble component in an amount ranging from about 5 wt % of the composite material to about 50 wt % of the composite material. For example, the soluble component may be present in the composite material in the amount that is about: 5-50 wt %, 10-45 wt %, 15-40 wt %, 20-40 wt %, 25-35 wt %, 5 wt %, 6 wt %, 7 wt %, 8 wt %, 9 wt %, 10 wt %, 11 wt %, 12 wt %, 13 wt %, 14 wt %, 15 wt %, 16 wt %, 17 wt %, 18 wt %, 19 wt %, 20 wt %, 21 wt %, 22 wt %, 23 wt %, 24 wt %, 25 wt %, 26 wt %, 27 wt %, 28 wt %, 29 wt %, 30 wt %, 31 wt %, 32 wt %, 33 wt %, 34 wt %, 35 wt %, 36 wt %, 37 wt %, 38 wt %, 39 wt %, 40 wt %, 41 wt %, 42 wt %, 43 wt %, 44 wt %, 45 wt %, 46 wt %, 47 wt %, 48 wt %, 49 wt %, or 50 wt % of the composite material. In some instances, however, the soluble component of the composite material may range from about 50 wt % of the composite material to about 95 wt % of the composite material.

In some cases, the composite material may comprise the soluble component in an amount that is no more than about 50 wt % of the composite material. For example, the soluble component may be present in the composite material in the amount that is no more than about: 5 wt %, 6 wt %, 7 wt %, 8 wt %, 9 wt %, 10 wt %, 11 wt %, 12 wt %, 13 wt %, 14 wt %, 15 wt %, 16 wt %, 17 wt %, 18 wt %, 19 wt %, 20 wt %, 21 wt %, 22 wt %, 23 wt %, 24 wt %, 25 wt %, 26 wt %, 27 wt %, 28 wt %, 29 wt %, 30 wt %, 31 wt %, 32 wt %, 33 wt %, 34 wt %, 35 wt %, 36 wt %, 37 wt %, 38 wt %, 39 wt %, 40 wt %, 41 wt %, 42 wt %, 43 wt %, 44 wt %, 45 wt %, 46 wt %, 47 wt %, 48 wt %, 49 wt %, or 50 wt % of the composite material.

In a non-limiting example, the composite material comprises about 70% wt insoluble component (e.g., TPU) and about 30% wt soluble component (e.g., PVA). In other non-limiting examples, the composite material may comprise about 90% wt insoluble component (e.g., TPU) and about 10% wt soluble component (e.g., PVA), about 80% wt insoluble component (e.g., TPU) and about 20% wt soluble component (e.g., PVA), about 60% wt insoluble component (e.g., TPU) and about 40% wt soluble component (e.g., PVA), and about 50% wt insoluble component (e.g., TPU) and about 50% wt soluble component (e.g., PVA). Such examples are non-limiting, and other ratios of the insoluble component to the soluble component may be used without departing from the scope of the present disclosure.

In some cases, the three-dimensional tissue scaffold is a heterogeneous construct fabricated from a plurality of materials. For example, each region may be fabricated from a different material. In some cases, at least one region may be fabricated from a composite material including an insoluble component and a soluble component. In some cases, the additional regions may be fabricated from a material other than the composite material. Examples of additional materials that may be used to fabricate a tissue scaffold are known in the art and may include, without limitation, polylactic acid (PLA), poly-L-lactic acid (PLLA), polyglycolic acid (PLGA), polycaprolactone (PCL), polydioxanone (PDO), collagen, fibrin and derivatives thereof, polysaccharides (e.g., chitosan), glycosaminoglycans (e.g., hyaluronic acid), fibrinogen, or gelatin. In some cases, the additional regions may be fabricated from a different composite material, such as a composite material having different insoluble and/or soluble components.

The three-dimensional tissue scaffold may be a homogeneous construct fabricated from a single material. For example, the tissue scaffold may be fabricated from a single material or from a composite material having the same insoluble and soluble components. In one example, each region may be fabricated from the same composite material (i.e., having the same soluble and insoluble components). In another example, each region may be fabricated with a different composition of the same composite material. For example, each region may be fabricated with a composite material having the same soluble and insoluble components, however, each region may have a different amount of soluble and insoluble components. By way of example only, one region may be fabricated from a composite material having about 70 wt % of insoluble component and about 30 wt % of soluble component, and another region may be fabricated from a composite material having about 50 wt % of the same insoluble component and about 50 wt % of the same soluble component. Without wishing to be bound by theory, varying the ratios of insoluble component to soluble component may alter the micro- and/or nano-structure (e.g., size of surface pits) of the scaffold. In this way, tissue scaffolds may be designed with different regions or regions that correspond to different tissue and/or cell types. For example, neuronal cells have been documented to respond to nano-scale pores and channels of from about 10 nm to about 30 nm, while osteoblasts respond to pores of from about 50 nm to about 100 nm. These pores sizes and distributions may be modulated by adjusting the composition percentage of soluble (temporary) to insoluble (permanent) material.

The three-dimensional tissue scaffold of the disclosure may be porous, for example, the tissue scaffold may comprise a plurality of pores. The plurality of pores may have any number or variety of shapes. For example, the pores may have a hexagonal, polygonal, circular, square, rectangular, and triangular shape. Each of the plurality of pores may have a pore width (and collectively may have an average pore width). In some cases, the plurality of pores may be fabricated in anyone, or combination of, a voronoi structure and/or a pre-designed geometric pattern including a plurality of sides and angles, as described further throughout. Pore widths and/or heights may range in size from about 1 µm to about 5 mm, for example from about 1 µm to about 50 µm, from about 10 µm to about 100 µm, from about 50 µm to about 250 µm, from about 150 µm to about 500 µm, from about 650 µm to about 1 mm, or from about 1 mm to about 5 mm. For example, pores may be about: 1 µm, 5 µm, 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm, 150 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, 1 mm, 1.1 mm, 1.2 mm, 1.3 mm, 1.4 mm, 1.5 mm, 1.6 mm, 1.7 mm, 1.8 mm, 1.9 mm, 2 mm, 2.1 mm, 2.2 mm, 2.3 mm, 2.4 mm, 2.5 mm, 2.6 mm, 2.7 mm, 2.8 mm, 2.9 mm, 3 mm, 3.1 mm, 3.2 mm, 3.3 mm, 3.4 mm, 3.5 mm, 3.6 mm, 3.7 mm, 3.8 mm, 3.9 mm, 4 mm, 4.1 mm, 4.2 mm, 4.3 mm, 4.4 mm, 4.5 mm, 4.6 mm, 4.7 mm, 4.8 mm, 4.9 mm, or 5 mm in width and/or height. Pore sizes may vary throughout the tissue scaffold, and may be determined by e.g., the rapid prototyping technique employed during fabrication of each region, or the pattern in which the regions are printed. Dissolution of the soluble component from each region may further superimpose a micro- or nanoporosity on a surface (e.g., plurality of pits on a fiber of the insoluble component) as further described herein.

The three-dimensional tissue scaffold of the disclosure may comprise a first region having a plurality of first pores with a first average pore width. The three-dimensional tissue scaffold may further comprise a second region having a plurality of second pores with a second average pore width. In some cases, the first average pore width and the second average pore width are different. In some cases, a three-dimensional tissue scaffold of the disclosure may further comprise a third region having a plurality of third pores, each comprising a third average pore width. In some cases, the third average pore width may be different from the first average pore width, the second average pore width, or both. In some cases, the first average pore width, the second average pore width, and the third average pore width are the same or substantially the same.

The three-dimensional tissue scaffolds may comprise two or more regions having an increasingly larger average pore width. For example, a three-dimensional tissue scaffold may have a first region having a plurality of pores of a first average pore width and a second region having a plurality of pores of a second average pore width, wherein the second average pore width is greater than the first average pore width. In some instances, a three-dimensional tissue scaffold may further have a third region having a plurality of pores of a third average pore width, wherein the third average pore width is greater than the first and the second average pore widths. In some cases, each region of the tissue scaffold may be fabricated to match a porosity associated with a tissue type (e.g., bone, cartilage, etc.). At least one region may be fabricated with a composite material having an insoluble and soluble component.

The three-dimensional tissue scaffold may comprise a plurality of pores, wherein a pore width of the plurality of pores changes in size along a gradient from a first region to a second region. In other words, a device may have discrete regions that change pore size in succession, or the size of the pores may change very gradually from a starting size to an ending size, based on a linear or non-linear change. This formation of a gradient may be an important mechanism for stem cell development and new tissue growth, especially for skeletal tissue and complex tissue. In some cases, the first region and second region are regions within a first region and a second region, respectively. In some cases, the first region and second region are fabricated from different materials. In other cases, the first region and second region are regions within the same region. In some cases, the gradient may be described by a linear function. In other cases, the gradient may be described by a step function. The step function may have at least 3 steps, for example, at least 3 steps, at least 4 steps, at least 5 steps, at least 6 steps, at least 7 steps, at least 8 steps, at least 9 steps, or at least 10 steps. In other cases, the gradient may be described by a sigmoidal function.

Each region of the tissue scaffold may have a different average pore width. In some cases, each region may have the same or a similar average pore width. In some cases, each region of the tissue scaffold may comprise the same material. In other cases, each region of the tissue scaffold may comprise a different material. In some embodiments, at least one region of the tissue scaffold may be fabricated with a composite material having an insoluble component and a soluble component. In some cases, each region of the tissue scaffold is fabricated using the same rapid prototyping technique. In other cases, each region of the tissue scaffold is fabricated using a different rapid prototyping technique. The tissue scaffold may have any number of regions. For example, the tissue scaffold may have 1, 2, 3, 4, 5, 6, 7, 8, 9, 10 or more than 10 regions.

In some cases, a tissue scaffold of the disclosure may have one or more regions, each region having a thickness. In some cases, a thickness of the region may be from about 0.1 mm to about 5 mm. For example, a region may have a thickness of about: 0.1 mm, 0.2 mm, 0.3 mm, 0.4 mm, 0.5 mm, 0.6 mm, 0.7 mm, 0.8 mm, 0.9 mm, 1.0 mm, 1.1 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, 2.4 mm, 2.5 mm, 2.6 mm, 2.7 mm, 2.8 mm, 2.9 mm, 3.0 mm, 3.1 mm, 3.2 mm, 3.3 mm, 3.4 mm, 3.5 mm, 3.6 mm, 3.7 mm, 3.8 mm, 3.9 mm, 4.0 mm, 4.1 mm, 4.2 mm, 4.3 mm, 4.4 mm, 4.5 mm, 4.6 mm, 4.7 mm, 4.8 mm, 4.9 mm, or 5.0 mm.

The three-dimensional tissue scaffold may be fabricated in any desired shape and size. In some cases, the tissue scaffold will be designed to match the shape and size of a tissue defect. In other cases, the tissue scaffold may be designed to match the shape and size of a surgical incision. In one example, the three-dimensional tissue scaffold is a hollow cylinder or has a cylindrical shape. In another example, the tissue scaffold is a non-circular cylinder (e.g., has an elliptical cross-section). In yet another example, the tissue scaffold is an irregular shape (e.g., designed to match an irregular shape of a tissue defect). In another example, the tissue scaffold has a shape customized to match the shape of a tissue defect. The tissue scaffold may further comprise a size that matches the size of a tissue defect. The tissue scaffold may, for example, have a size ranging from about 1 to about 4 mm in thickness, and about 6 mm to about 2 cm in diameter.

In some embodiments, the porosity and/or density of the tissue scaffold may be designed to correspond with the porosity and/or density of the tissue in which the tissue scaffold will be implanted. For example, if the tissue scaffold is to be implanted into a tissue defect involving bone, the tissue scaffold may have a porosity and/or density that is similar to or the same as bone. Often, the tissue defect will involve a complex arrangement of multiple different tissue and cell types, such that the tissue scaffold may be designed to have multiple regions or regions that correspond to the multiple different tissue and cell types. In some cases, a single region of the tissue scaffold fabricated from a single material (e.g., a composite material), may be capable of supporting the growth and regeneration of multiple different cell types.

In a non-limiting example, a three-dimensional tissue scaffold may have a first region having a plurality of first pores, wherein an average pore width of the plurality of first pores is from 0 to about 50 µm. For example, the average pore size of the first region may be about: 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, or 50 µm. In another example, a tissue scaffold may further have a second region attached to a side of the first region and having a plurality of second pores, wherein an average pore width of the plurality of second pores is from about 100 µm to about 200 µm. For example, the average pore size of a second region may be about: 100 µm, 110 µm, 120 µm, 130 µm, 140 µm, 150 µm, 160 µm, 170 µm, 180 µm, 190 µm, or about 200 µm. In another example, a tissue scaffold may further have a third region attached to a side of the second region and having a plurality of third pores, wherein an average pore width of the plurality of third pores is from about 500 µm to about 1 mm. For example, the average pore size of a third region may be about: 500 µm, 550 µm, 600 µm, 650 µm, 700 µm, 750 µm, 800 µm, 850 µm, 900 µm, 950 µm, or 1 mm. In some cases, at least one of the first region, the second region, or the third region is fabricated with a composite material having an insoluble component and a soluble component.

The three-dimensional tissue scaffold may be exposed to or immersed in a solvent that is capable of dissolving the soluble component from the scaffold such that the insoluble component remains in the scaffold. In preferred cases, the insoluble component remains at least partially intact (e.g., the same shape and/or size) as fabricated in the scaffold, after dissolution of the soluble component. The soluble component may be dissolved from the tissue scaffold prior to use. Dissolution of the soluble component can be performed by the manufacturer and sold to an end-user (e.g., a physician), or may be performed by an end-user. In some cases, the tissue scaffold is sold (e.g., in a kit) or otherwise provided to an end-user after dissolution of the soluble component.

In some cases, dissolution of the soluble component may generate a plurality of pits along a surface of the insoluble material. In a non-limiting example in which a tissue scaffold is fabricated using TPU/PVA, the tissue scaffold may be immersed in or otherwise contacted with water to dissolve the PVA component, such that the TPU component remains essentially intact in the shape and/or size as it was originally fabricated, however, the remaining insoluble component may comprise a plurality of pits along a surface (e.g., of a fiber). Without wishing to be bound by theory, the plurality of pits may create a unique stratified micro- and/or nano- structure that is capable of supporting the growth of a plurality of different cell types and the regeneration of a plurality of different tissue types. In some cases, the plurality of pits generated by dissolution of the soluble component may have a size (e.g., an average width) that is below the resolution of a rapid prototyping technology (e.g., below 50 µm). The plurality of pits generated by dissolution of the soluble component may have a size ranging from about 1 nm to about 50 µm, for example, from about 1 nm to about 10 nm, from about 5 nm to about 50 nm, from about 50 nm to about 150 nm, from about 125 nm to about 200 nm, from about 150 nm to about 500 nm, from about 250 nm to about 1 µm, from about 750 nm to about 5 µm, from about 5 µm to about 25 µm, or from about 10 µm to about 50 µm. For example, the plurality of pits may have a size of about: 1 nm, 10 nm, 20 nm, 30 nm, 40 nm, 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, 1 µm, 5 µm, 10 µm, 15 µm, 20 µm, 25 µm, 30 µm, 35 µm, 40 µm, 45 µm, or 50 µm. In some cases, the size of the pits is below the resolution of standard three-dimensional printers.

The three-dimensional tissue scaffolds of the disclosure may have an elastic modulus, after dissolution of the soluble component, ranging from about 1 MPa to about 1000 MPa, from example, from about 1 MPa to about 50 MPa, from about 50 MPa to about 500 MPa, from about 50 MPa to about 100 MPa, from about 100 MPa to about 500 MPa, from about 250 MPa to about 750 MPa, or from about 500 MPa to about 1000 MPa. For example, the elastic modulus of a tissue scaffold, after dissolution of the soluble component, may be about: 1 MPa, 5 MPa, 10 MPa, 25 MPa, 50 MPa, 75 MPa, 100 MPa, 125 MPa, 150 MPa, 175 MPa, 200 MPa, 225 MPa, 250 MPa, 275 MPa, 300 MPa, 325 MPa, 350 MPa, 375 MPa, 400 MPa, 425 MPa, 450 MPa, 475 MPa, 500 MPa, 525 MPa, 550 MPa, 575 MPa, 600 MPa, 625 MPa, 650 MPa, 675 MPa, 700 MPa, 725 MPa, 750 MPa, 775 MPa, 800 MPa, 825 MPa, 850 MPa, 875 MPa, 900 MPa, 925 MPa, 950 MPa, 975 MPa, or 1000 MPa. In some cases, the elastic modulus of the tissue scaffold, after dissolution of the soluble component, is similar to that of calcified cartilage.

The tissue scaffold may be coated prior to implantation of the scaffold into a subject. Non-limiting examples of coatings that may be employed include nano-hydroxyapatite (nHa) coatings, tricalcium phosphate (TCP) coatings, and antibacterial metal-based nanoparticles. In some cases, the tissue scaffold is nucleated prior to implantation, by, e.g., incubating in a simulated bodily fluid (SBF), to promote bone regeneration. The three-dimensional tissue scaffolds may further be coated with any number of bioactive factors that may promote growth and/or regeneration of tissue. Non-limiting examples may include: growth factors, hormones, morphogenetic factors, such as bone morphogenic protein (BMP) and derivatives thereof, vascular endothelial growth factor (VEGF) and derivatives thereof, transformative growth factor (TGF) and derivatives thereof, as well as any amine linkage or amino acid group isolated from or intended to replicate specific portions of these growth factors.

The tissue scaffolds as described herein may be manufactured from a rapid prototyping technology including, but not limited to, stereolithography (SLA), digital light processing (DLP), fused deposition modeling (FDM), selective laser sintering (SLS), selective laser melting (SLM), electron beam melting (EBM), laminated object manufacturing (LOM), and the like.

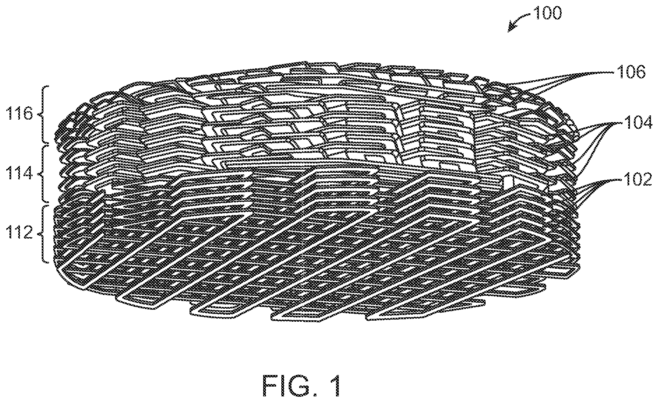

Reference is now made to the drawings and embodiments of the disclosure. FIG. 1 depicts a bottom perspective view of an exemplary tissue scaffold 100 according to one or more embodiments of the disclosure provided herein. Tissue scaffold 100 of FIG. 1 is an illustrative and non-limiting example and is exhibited for the purposes of discussing the specificities of the tissue scaffolds contemplated in this disclosure. One of ordinary skill in the art will readily appreciate that the specificities discussed below in regard to tissue scaffold 100 may be incorporated into tissue scaffolds corresponding to defects of any of a plurality of dimensions and corresponding to any of a plurality of bone, cartilage, and/or osteochondral areas. Furthermore, it is to be understood that all disclosures of tissue scaffolds provided herein in regard to embodiments including, but not limited to, porosity, pore number, pore sizing, pore shape, elastic modulus, coatings, pits, pit number, pit sizing, pit shape, material composition, and the like may be applicable to the discussion provided below in regard to the tissue scaffold 100 of FIG. 1.

Tissue scaffold 100 may be an arthroscopically implantable three-dimensional tissue scaffold and may be comprised of a plurality of regions. For example, as shown in FIG. 1, tissue scaffold 100 may include a first region 112, a second region 114, and a third region 116. Each of regions 112, 114, and 116 may be formed from one or more layers. For instance, region 112 may be formed from one or more first layers 102, region 114 may be formed from one or more second layers 104, and region 116 may be formed from one or more third layers 106. In some instances, tissue scaffold 100 may include a fewer number of regions (e.g., one or two regions) or a greater number of regions (e.g., three or more regions). Similarly, tissue scaffold 100 may include a fewer number a layers per region or a greater number of layers per region. In some instances, tissue scaffold 100 may be designed to facilitate tissue regeneration across one or more types of tissues.

Layers 102, 104, and 106 of regions 112, 114, and 116 of tissue scaffold 100 may be composed of a first material comprising at least an insoluble component and a soluble component. As an illustrative and non-limiting example, the first material used to fabricate layers 102, 104, and 106 of regions 112, 114, and 116 of tissue scaffold 100 may be a TPU/PVA composite such as Gel-lay or any other material which comprises at least a soluble component and an insoluble component. Alternatively, the first material may consist of a soluble component and an insoluble component such as any of the materials described herein which exhibit the properties of solubility and insolubility, as well as any other such materials.

In some instances, different ratios of the insoluble component to the soluble component of the first material may be used in layers 102, 104, and 106. As an illustrative and non-limiting example, first layers 102 may be formed from the first material having a first ratio of the insoluble component to the soluble component, second layers 104 may be formed from the first material having a second ratio of the insoluble component to the soluble component, and third layers 106 may be formed from the first material having a third ratio of the insoluble component to the soluble component. Accordingly, first region 112 may be formed from the first ratio of the first material, region 114 may be formed from the second ration of the first material, and region 116 may be formed from the third ratio of the first material. In some cases, the ratio of the insoluble component to the soluble component of the first material may change on a layer-by-layer basis within any given region.

Layers 102, 104, and 106 of regions 112, 114, and 116 of tissue scaffold 100 may alternatively be composed of a material (e.g., a second material) other than the first material. The second material used to fabricate layers 102, 104, and 106 of regions 112, 114, and 116 may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein. Accordingly, any multitude of materials (e.g., a third material, a fourth material, and so on) may be used to fabricate layers 102, 104, and 106 of regions 112, 114, and 116. The materials used, and ratios of components in materials, may change on a region-by-region basis, layer-by-layer basis within regions, and/or any combination thereof.

First region 112 of tissue scaffold 100 may be formed from one or more layers 102 (e.g., first layers). Each of the one or more layers 102 may correspond to a material deposit instance from a rapid prototyping technology at a particular vertical step and/or increment. In some instances, one or more layers 102 may be formed in a serpentine and/or sinusoidal pattern around a constraining boundary, such as a circle, oval, square, and/or any other regular or irregular geometric shape. In some instances, the serpentine and/or sinusoidal pattern may correspond to a continuous, semi-continuous, or noncontinuous in-fill pattern of a rabid prototyping technology.

In forming first region 112 of tissue scaffold 100, the serpentine and/or sinusoidal pattern of the one or more first layers 102 may rotate on a layer-by-layer basis. For example, a first layer of the one or more layers 102 may be of a serpentine and/or sinusoidal pattern at a first rotation and/or rotational offset, a second layer of the one or more layers 102 may be of a serpentine and/or sinusoidal pattern at a second rotation and/or rotational offset, a third layer of the one or more layers 102 may be of a serpentine and/or sinusoidal pattern at a third rotation and/or rotational offset, and so on.

Second region 114 of tissue scaffold 100 may be formed from one or more layers 104 (e.g., second layers). Each of the one or more layers 104 may correspond to a material deposit instance from a rapid prototyping technology at a particular vertical step and/or increment. In some instances, one or more layers 104 may also be formed in a serpentine and/or sinusoidal pattern around a constraining boundary, such as a circle, oval, square, and/or any other regular or irregular geometric shape. In forming second region 114 of tissue scaffold 100, the serpentine and/or sinusoidal pattern of the one or more second layers 104 may rotate on a layer-by-layer basis. For example, a first layer of the one or more layers 104 may be of a serpentine and/or sinusoidal pattern at a third rotation and/or rotational offset, a second layer of the one or more layers 104 may be of a serpentine and/or sinusoidal pattern at a fourth rotation and/or rotational offset, a third layer of the one or more layers 104 may be of a serpentine and/or sinusoidal pattern at a fifth rotation and/or rotational offset, and so on.

Third region 116 of tissue scaffold 100 may be formed from one or more layers 106 (e.g., third layers). Each of the one or more layers 106 may correspond to a material deposit instance from a rapid prototyping technology at a particular vertical step and/or increment. In some instances, one or more layers 106 may also be formed in a serpentine and/or sinusoidal pattern around a constraining boundary, such as a circle, oval, square, and/or any other regular or irregular geometric shape. In forming third region 116 of tissue scaffold 100, the serpentine and/or sinusoidal pattern of the one or more third layers 106 may rotate on a layer-by-layer basis. For example, a first layer of the one or more layers 106 may be of a serpentine and/or sinusoidal pattern at a sixth rotation and/or rotational offset, a second layer of the one or more layers 106 may be of a serpentine and/or sinusoidal pattern at a seventh rotation and/or rotational offset, a third layer of the one or more layers 106 may be of a serpentine and/or sinusoidal pattern at an eighth rotation and/or rotational offset, and so on.

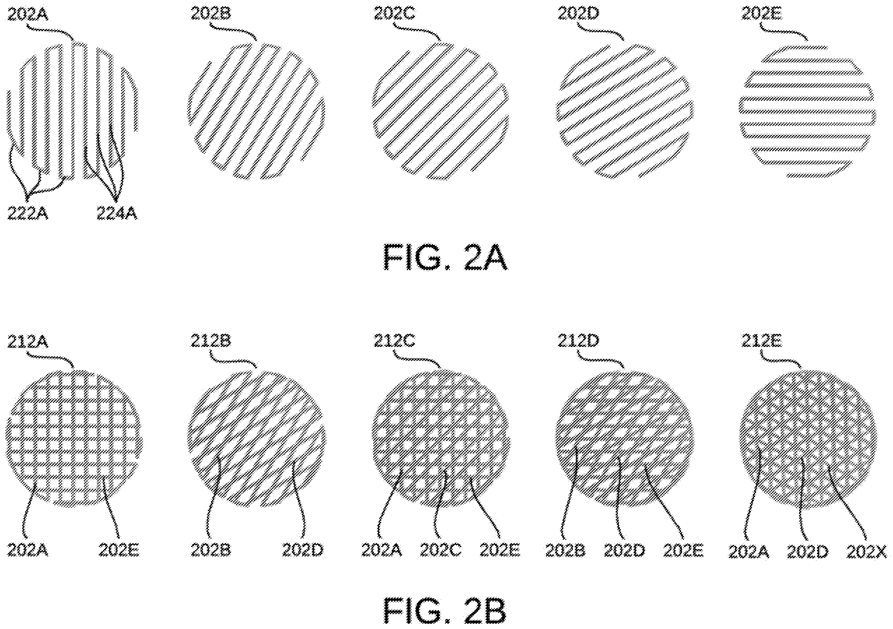

To provide further details regarding the layers, rotations, and rotational offsets of layers in forming regions of tissue scaffolds, reference is now made to FIG. 2A, which depicts a plurality of individual layers of an exemplary tissue scaffold according to one or more embodiments of the present disclosure.

Layer 202A of FIG. 2A depicts an exemplary layer used in forming an exemplary region of an exemplary tissue scaffold. The material deposit of layer 202A may be of a serpentine and/or sinusoidal pattern, and may include a plurality of boundary segments 222A and a plurality of crossing segments 224A. Each of the plurality of boundary segments 222A may take the form of a boundary region corresponding to the shape of the layer of the tissue scaffold. For example, in the event that the layer of the tissue scaffold has a circular shape, each of the plurality of boundary segments 222A may form a circular shape in combination. Similarly, in the event that the layer of the tissue scaffold has an oval shape, each of the plurality of boundary segments 222A may form an oval shape in combination. Further, in the event that the layer of the tissue scaffold has a square shape, each of the plurality of boundary segments 222A may form a square shape in combination. As such, each of the plurality of boundary segments 222A may take a shape corresponding to that of the shape of the layer of the tissue scaffold.

**The** plurality of crossing segments 224A may extend across layer 202A from boundary segments 222A on opposing sides of layer 202A. Depending on the shape of layer 202A, crossing segments 224A may be of a same length, or differing lengths. For example, in the event that the layer of the tissue scaffold has a circular shape, crossing segments 224A may have differing lengths depending on where the circular shaped layer is being traversed. Specifically, crossing segments 224A near a distal portion of the circular shaped layer may have shorter lengths than crossing segments 224A near the center portion of the circular shaped layer. Conversely, in the event that the layer of the tissue scaffold has a square shape, crossing segments 224A may have a same length.

While a number of boundary segments 222A and crossing segments 224A are shown, it may be understood that the number of boundary segments 222A and crossing segments 224A may be increased or decreased and thereby change the total volume of deposited material of layer 202A. For example, if the shape and size of layer 202A are consistent, an increase in the number of boundary segments 222A and crossing segments 224A may correspond to an increase in the total volume of deposited material. Conversely, if the shape and size of layer 202A are consistent, a decrease in the number of boundary segments 222A and crossing segments 224A may correspond to a decrease in the total volume of deposited material. As such, by increasing and/or decreasing the number of boundary segments 222A and crossing segments 224A, layer 202A may be tailored to meet the structural and mechanical characteristics of native tissue.

In addition to layer 202A, FIG. 2A also depicts layers 202B, 202C, 202D, and 202E. The additional layers are similar to layer 202A, but are presented at different degrees of rotation relative to a common frame of reference. For example, layer 202A is at a first rotation (e.g., 90 degrees), layer 202B is at a second rotation (e.g., 60 degrees), layer 202C is at a third rotation (e.g., 45 degrees), layer 202D is at a fourth rotation (e.g., 20 degrees), and layer 202E is at a fifth rotation (e.g., 0 degrees). While layers 202A, 202B, 202C, 202D, and 202E are presented at particular rotations from a common frame of reference, such rotations are non-limiting and for illustrative purposes, and any amount of rotation between 0 degrees and 360 degrees is within the scope of the present disclosure.

In some cases, the rotations of layers may be described as rotational offsets in relation to other layers. For example, layer 202A may be at a first rotational offset from layer 202E (e.g., 270 degrees), layer 202B may be at a second rotational offset from layer 202A (e.g., 30 degrees), layer 202C may be at a third rotational offset from layer 202A (e.g., 45 degrees), layer 202D may be at a fourth rotational offset from layer 202A (e.g., 60 degrees), and layer 202E may be at a fifth rotational offset from layer 202A (e.g., 90 degrees). While layers 202A, 202B, 202C, 202D, and 202E are presented at particular rotational offsets relative to other layers, such rotations are non-limiting and for illustrative purposes, and any amount of rotational offset between 0 degrees and 360 degrees is within the scope of the present disclosure.

To form regions of a tissue scaffold, layers 202A, 202B, 202C, 202D, and 202E may be deposited alone, or in combination, across a plurality of vertical steps and/or increments. For example, in regard to FIG. 2B, region 212A may be formed from a combination of layers 202A and 202E, region 212B may be formed from a combination of layers 202B and 202D, region 212C may be formed from a combination of layers 202A, 202C, and 202E, region 212D may be formed from a combination of layers 202B, 202D, and 202E, and region 212E may be formed from a combination of layers 202A, 202D, and a third layer, 202X, which may be at rotation of 120 degrees relative to the frame of reference of layers 202A and 202D. While regions 212A, 212B, 212C, 212D, and 212E are presented as formed by particular combinations of layers 202A, 202B, 202C, 202D, and 202E, such regions are non-limiting and for illustrative purposes, and any combination of layers of any amount of rotation between 0 degrees and 360 degrees may be used to form regions of a tissue scaffold without departing from the scope of the present disclosure.

In forming regions 212A, 212B, 212C, 212D, and 212E, layers 202A, 202B, 202C, 202D, and 202E may be applied sequentially by a rapid prototyping technology until the region in complete. For example, in the case of region 212A, layers 202A and 202E may be deposited on an alternating basis by a rapid prototyping technology until region 212A is complete. Specifically, as an illustrative and non-limiting example, layer 202A may be deposited first, then layer 202E, followed by layer 202A again, then layer 202E again, and so on until region 212A is complete. Similarly, in the case of region 212C, layers 202A, 202C, and 202E may be deposited on an alternating basis until region 212C is complete. Specifically, as an illustrative and non-limiting example, layer 202A may be deposited first, then layer 202C, followed by layer 202E, then layer 202A again, followed by layer 202E again, and then layer 202C again, and so on until region 212C is complete. The layer-by-layer deposition process may be similar for regions 212B, 212D, and 212E, as well as all other possible combinations of layers in formation of corresponding regions.

In some instances, layers 202A, 202B, 202C, 202D, and 202E may be deposited one or more times at a corresponding number of vertical steps and/or increments by a rapid prototyping technology before a subsequent layer is deposited in forming a region of a tissue scaffold. For example, in forming region 212B, layer 202B may be deposited one or more times at a corresponding one or more vertical steps and/or increments. Specifically, as an illustrative and non-limiting example, in the event that layer 202B is deposited three times before proceeding to layer 202D, a first deposition of layer 202B may be deposited at a first vertical step and/or increment, a second deposition of layer 202B may be deposited at a second vertical step and/or increment, and third deposition of layer 202B may be deposited at a third vertical step and/or increment before commencing with deposition of layer 202D at a fourth vertical step and/or increment. The layer-by-layer deposition process may be similar for regions 212A, 212C, 212D, and 212E, as well as all other possible combinations of layers in formation of corresponding regions. In doing so, layers may be deposited any number of times at any number of vertical steps and/or increments in formation of corresponding regions.

In other instances, the number of depositions of layers may differ between different layer types. For instance, as an illustrative and non-limiting example, in forming region 212D, layer 202B may be deposited three times at three steps and/or increments, layer 202D may be deposited five times at five steps and/or increments, and layer 202E may be deposited seven times at seven steps and/or increments. The differing amounts of layer depositions across different layer types may be similar for regions 212A, 212C, 212D, and 212E, as well as all other possible combinations of layers in formation of corresponding regions.

The deposition of layers may change on layer instance by layer instance basis. For instance, as an illustrative and non-limiting example, in forming region 212E, layer 202A may be deposited three times during a first deposition instance, seven times at a second deposition instance, and two times during a third deposition instance. The changing amount of layer depositions on a layer instance by layer instance basis may be similar for regions 212A, 212C, 212D, and 212E, as well as all other possible combinations of layers and corresponding regions.

In some instances, layers 202A, 202B, 202C, 202D, and 202E of FIG. 2A and combinations thereof in forming regions 212A, 212B, 212C, 212D, and 212E of FIG. 2B, may increase the foldability, deformability, and/or otherwise the rotational pliability along an axis parallel to a front face of the resulting tissue scaffold. In some cases, in forming the tissue scaffold, each of the layers, such as layers 202A, 202B, 202C, 202D, and 202E, may be rotated at acute angles (e.g., less than 90 degrees) on a layer-by-layer basis. The rotation of layers may further occur across all regions of the tissue scaffold such that the rotational offset between each layer of the tissue scaffold is at an acute angle. The angle of rotation between each of the layers of the scaffold may be acute, but may vary on a layer-by-layer basis. By doing so, the resulting tissue scaffold may display increased foldability, deformability, and/or otherwise the rotational pliability along an axis parallel to a front face of the resulting tissue scaffold. The mechanical properties may enable the scaffold to be rolled along the axis parallel to the front face of the scaffold such that the scaffold, when rolled, may be able to be fitted into an arthroscopic tool for surgical insertion into the tissue defect area. Other angles between layers and/or regions are contemplated herein such as angles equal to 90 degrees (e.g., right angles), or angles greater than 90 degrees (e.g., obtuse angles).

While layers 202A, 202B, 202C, 202D, and 202E of FIG. 2A are of serpentine and/or sinusoidal pattern, more complex patterns are achievable without departing from the scope of the present disclosure. In some instances, geometries corresponding to regions 212A, 212B, 212C, 212D, and 212E of FIG. 2B are achievable as individual layers, rather than combinations of layers. Accordingly, region 212A may be a single layer, region 212B may be a single layer, region 212C may be a single layer, region 212D may be a single layer, and region 212E may be a single layer. Thus, any geometric pattern is achievable for any given layer within a region of a tissue scaffold without departing from the scope of the present disclosure.

Layers 202A, 202B, 202C, 202D, and 202E of FIG. 2A and combinations thereof in forming regions 212A, 212B, 212C, 212D, and 212E of FIG. 2B, may contribute to the porosity of different regions of tissue scaffolds. For example, in the event that each layer is the same aside from having a different rotation relative to a common frame of reference, a region composed of one layer will have a higher porosity than a region of two layers. Similarly, a region having two layers will have a higher porosity than a region having three layers. In this way, by combining a greater or fewer number of layers each of which at differing rotations, an increase or decrease in porosity can be achieved. As an illustrative and non-limiting example, region 212A composed from two layers at different rotations (e.g., layers 202A and 202E) may have a higher porosity than region 203E composes from three layers at different rotations (e.g., layers 202A, 202D, and 202X).

Additionally and/or alternatively, by altering the number of boundary segments, crossing segments, and total volume of material deposited on a layer-by-layer basis, the porosity of the corresponding region may be increased or decreased. Specifically, as an illustrative and non-limiting example, an increase in the number of boundary segments, crossing segments, and/or total volume of material deposited on a layer-by-layer basis, the porosity of the layers and/or corresponding region may decrease. Conversely, a decrease in the number of boundary segments, crossing segments, and/or total volume of material deposited on a layer-by-layer basis, the porosity of the layers and/or corresponding region may increase.

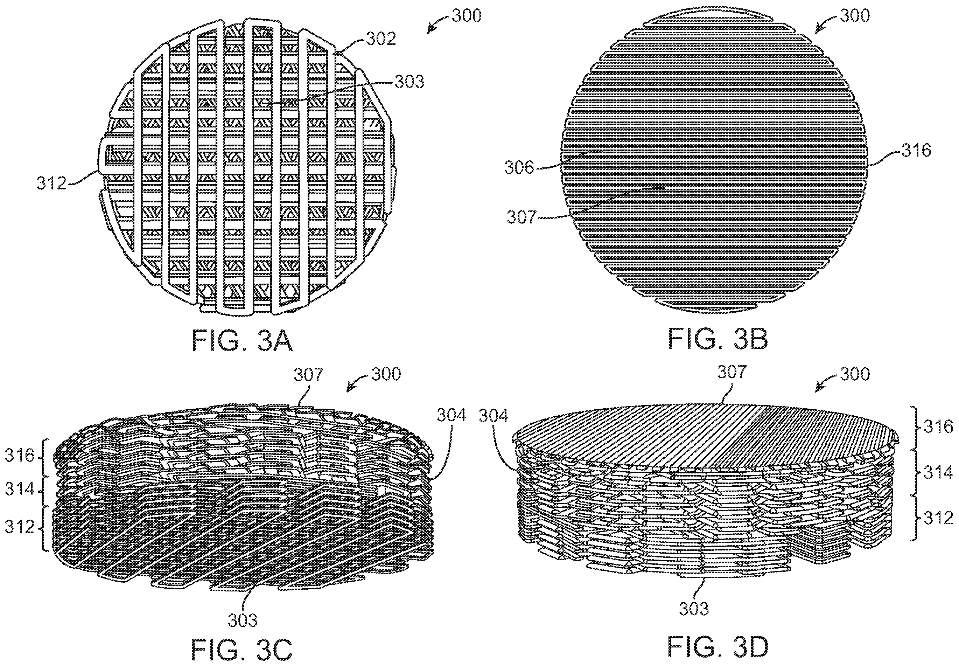

Attention is now drawn to FIGS. 3A, 3B, 3C, and 3D, which respectively depict a bottom view, a top view, a bottom perspective view, and a top perspective view of a first exemplary tissue scaffold 300 according to one or more embodiments of the disclosure provided herein. The tissue scaffold 300 of FIGS. 3A, 3B, 3C, and 3D is an illustrative and non-limiting example and is exhibited for the purposes of discussing the specificities of the tissue scaffolds contemplated in this disclosure. One of ordinary skill in the art will readily appreciate that the specificities discussed below in regard to tissue scaffold 300 may be incorporated into tissue scaffolds corresponding to defects of any of a plurality of dimensions and corresponding to one or more of bone, cartilage, and/or osteochondral areas. Furthermore, it is to be understood that all disclosures of tissue scaffolds provided herein in regard to embodiments including, but not limited to, porosity, pore number, pore sizing, pore shape, elastic modulus, coatings, pits, pit number, pit sizing, pit shape, material composition, and the like may be applicable to the discussion provided below in regard to the tissue scaffold 300 of FIGS. 3A, 3B, 3C, and 3D. In some instances, tissue scaffold 300 may be designed to facilitate tissue regeneration across one or more types of tissues.

Tissue scaffold 300 may be an arthroscopically implantable three-dimensional tissue scaffold and may be comprised of a plurality of regions. For example, as shown in FIGS. 3A and 3B, tissue scaffold 300 may include a first region 312 and a third region 316. Further, as shown in FIGS. 3C and 3D, tissue scaffold 300 may also include a second region 314. Each of regions 312, 314, and 316 may be formed from one or more layers. For instance, region 312 may be formed from one or more layers 302 (e.g., first layers), region 314 may be formed from one or more layers 304 (e.g., second layers), and region 316 may be formed from one or more layers 306 (e.g., third layers). Alternatively, region 312 may be formed a plurality of layers 302 (e.g., first layers), region 314 may be formed a plurality of layers 304 (e.g., second layers), and region 316 may be formed from a plurality layers 306 (e.g., third layers). In some instances, tissue scaffold 300 may include a fewer number of regions (e.g., one or two regions) or a greater number of regions (e.g., three or more regions). Similarly, tissue scaffold 300 may include a fewer number of layers per region or a greater number of layers per region. Layers 302, 304, and 306 and/or regions 312, 314, and 316 of tissue scaffold 300 may respectively promote tissue growth of one or more of a first tissue, a transitionary tissue at a transitionary region between the first tissue and a second tissue, and the second tissue.

Layers 302, 304, and 306 of regions 312, 314, and 316 of tissue scaffold 300 may be composed of a first material comprising at least an insoluble component and a soluble component. As an illustrative and non-limiting example, the first material used to fabricate layers 302, 304, and 306 of regions 312, 314, and 316 of tissue scaffold 300 may be a TPU/PVA composite such as Gel-lay or any other material which comprises at least a soluble component and an insoluble component. Alternatively, the first material may consist of a soluble component and an insoluble component such as any of the materials described herein which exhibit the properties of solubility and insolubility, as well as any other such materials.

In some instances, different ratios of the insoluble component to the soluble component of the first material may be used in layers 302, 304, and 306. As an illustrative and non-limiting example, one or more first layers 302 may be formed from the first material having a first ratio of the insoluble component to the soluble component, one or more second layers 304 may be formed from the first material having a second ratio of the insoluble component to the soluble component, and one or more third layers 306 may be formed from the first material having a third ratio of the insoluble component to the soluble component. Accordingly, first region 312 may be formed from the first ratio of the first material, region 314 may be formed from the second ratio of the first material, and region 316 may be formed from the third ratio of the first material. In some cases, the ratio of the insoluble component to the soluble component of the first material may change on a layer-by-layer basis within any given region.

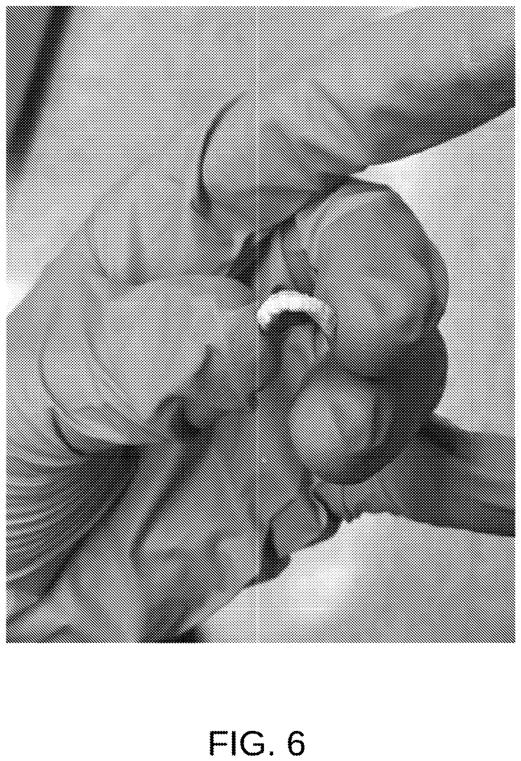

When initially manufactured, tissue scaffold 300 may have a first set of mechanical properties owing to the presence of both the insoluble component and the soluble component in the first material. However, after being exposed to a solvent of any of the types described herein and in any of the manners described herein, tissue scaffold 300 may have a second set of mechanical properties owing to the presence of only the insoluble component. In some cases, the first set of mechanical properties may correspond to rigidity and stiffness of tissue scaffold 300 after initial manufacture, whereas the second set of mechanical properties may correspond to flexibility and elasticity of tissue scaffold 300 after being treated by the solvent. For example, reference is now made to FIG. 6, which depicts an illustrative and non-limiting example of a tissue scaffold that may be implanted arthroscopically into an osteochondral defect according to one or more embodiments of the present disclosure. As shown in FIG. 6, the tissue scaffold may be flexible and elastic after being treated by the solvent.

Returning to FIGS. 3A, 3B, 3C, and 3D, layers 302, 304, and 306 of regions 312, 314, and 316 of tissue scaffold 300 may also and/or alternatively be composed of a material (e.g., a second material) other than the first material. The second material used to fabricate layers 302, 304, and 306 of regions 312, 314, and 316 may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein. Accordingly, any multitude of materials (e.g., a third material, a fourth material, and so on) may be used to fabricate layers 302, 304, and 306 of regions 312, 314, and 316. The materials used, and ratios of components in materials, may change on a region-by-region basis, layer-by-layer basis within regions, and/or any combination thereof.

Each of the one or more layers 302 of region 312 may correspond to a material deposit instance from a rapid prototyping technology at a particular vertical step and/or increment. In some instances, the one or more layers 302 may be formed in a serpentine and/or sinusoidal pattern around a constraining boundary, such as a circle, oval, square, and/or any other regular or irregular geometric shape. In forming the first region 312 of tissue scaffold 300, the serpentine and/or sinusoidal pattern of the one or more first layers 302 may rotate on a layer-by-layer basis. In some cases, each of the one or more layers 302 of region 312 may alternate rotations on a layer-by-layer basis, but may repeat rotations every two layers. As an illustrative and non-limiting example, a first layer of the one or more layers 302 may be of a first serpentine and/or sinusoidal pattern at a first rotation, a second layer of the one or more layers 302 may be of the first serpentine and/or sinusoidal pattern at a second rotation, and then a third layer of the one or more layers 302 may be of the first serpentine and/or sinusoidal pattern and may restart the rotation pattern at the first rotation.

In some instances, a first layer of one or more layers 302 may correspond to layer 202A of FIG. 2A, and a second layer of one or more layers 302 may correspond to layer 202E of FIG. 2A. As such, region 312 of tissue scaffold 300 may resemble region 212A of FIG. 2B. Alternatively, the one or more layers 302 of region 312 may be similar to layers 202B, 202C, 202D, 202E, or any other rotational displacement of layer 202A from 0 degrees to 360 degrees around a common frame of reference. Further, the one or more layers 302 of scaffold 300 may have greater or fewer number of the plurality of boundary segments 222A and the plurality of crossing segments 224A as provided in layer 202A. Accordingly, region 312 of the tissue scaffold 300 may be similar to any of regions 212A, 212B, 212C, 212D, 212E, or any other region as defined by any possible combination of layers. In some instances, the number of boundary and/or crossing segments of the one or more layers 302 may increase, decrease, or remain constant on a layer-by-layer basis in region 312. In other instances, each of the layers 302 (e.g., a first layer, a second layer, and so on) of region 312 may have a first number of boundary segments, a first number of crossing segments, and may be of the first sinusoidal pattern.

Region 312 of the tissue scaffold 300 may be designed to interface with a first native tissue, such as bone, and may be of a first porosity and have a first plurality of pores, each comprising a first average pore width. In some cases, the first porosity, first plurality of pores, and first average pore width may resemble the nano- and/or microstructure and/or topography of the first native tissue. Region 312 of the tissue scaffold 300 may be formed from a first ratio of a first material comprising at least the insoluble component and the soluble component, as described above. When the soluble component is dissolved in a given solvent, the insoluble component may remain and may have an altered nano- and/or micro- structure and/or topography. In some cases, the insoluble component may have a first plurality of pits corresponding to the dissolved soluble component. In some instances, the first ratio of the soluble component and the insoluble component of the first material may be such that the pitted nano- and/or micro- structure and/or topography resembles that of the first native tissue and promotes tissue regeneration of the first native tissue.

Region 312 of the arthroscopically implantable three-dimensional tissue scaffold 300 may be configured to be inserted completely below an outermost face of a surgically induced and/or natural tissue defect to the first native tissue. For example, in the case of microfracture surgery or other excavatory surgical techniques which cause a pocket or recess to be formed internally and/or inwardly from the outermost face of the first native tissue, region 312 of the tissue scaffold 300 may be configured to be situated in the pocket or recess such that a bottommost face 303 of region 312 of the tissue scaffold 300 is contacting a topmost face of the pocket or recess, and a topmost layer (e.g., last layer of one or more layers 302 of region 312 before transitioning to a first layer of one or more layers 304 of region 314) of region 312 of the tissue scaffold 300 is below, beneath, or at the same level of the outermost face of the first native tissue.

In some instances, region 312 may have a highest porosity, greatest number of pores, and/or largest average pore width in comparison to regions 314 and 316 of tissue scaffold 300. The porosity, number of pores, and average pore width of region 312 may encourage internal fluids and stem cells from an internal bone cavity expelled from a surgical treatment such as microfracture surgery upward and into tissue scaffold 300. Furthermore, the first material used in forming region 312 may have the highest ratio of the insoluble component to the soluble component in comparison to regions 314 and 316. In other cases, however, the first material used in forming region 312 may the lowest porosity, fewest number of pores, smallest average pore width, and/or have the lowest ratio of the insoluble component to the soluble component in comparison to regions 314 and 316.

Region 314 of the tissue scaffold may be designed to form a transitionary region between region 312 corresponding to the first native tissue (e.g., bone) and region 316 corresponding to a second native tissue (e.g., cartilage). Region 314 may be of a particular porosity and have a particular plurality of pores, each comprising a particular average pore width. For example, region 314 may be of a second porosity and have a second plurality of pores, each comprising a second average pore width.

Each of the one or more layers 304 of region 314 may correspond to a material deposit instance from a rapid prototyping technology at a particular vertical step and/or increment. In some instances, the one or more layers 304 may be formed in a serpentine and/or sinusoidal pattern around a constraining boundary, such as a circle, oval, square, and/or any other regular or irregular geometric shape. In forming the second region 314 of tissue scaffold 300, the serpentine and/or sinusoidal pattern of the one or more second layers 304 may rotate on a layer-by-layer basis. In some cases, each of the one or more layers 304 of region 314 may alternate rotations on a layer-by-layer basis, but may repeat rotations every three layers. As an illustrative and non-limiting example, a first layer of the one or more layers 304 may be of the first serpentine and/or sinusoidal pattern at the first rotation, a second layer of the one or more layers 304 may be of the first serpentine and/or sinusoidal pattern at a third rotation, a third layer of the one or more layers 304 may be of the first serpentine and/or sinusoidal pattern at the second rotation, and a fourth layer of the one or more layers 304 maybe of the first serpentine and/or sinusoidal pattern and may restart the rotation pattern at the first rotation.

In some cases, region 314 of the tissue scaffold 300 may include one or more layers 304. Each of the one or more layers 314 may be rotated from 0 degrees to 360 degrees around a common frame of reference in forming region 314 of tissue scaffold 300. In some instances, the one or more layers 304 of region 314 may be similar to any of layers 202A, 202B, 202C, 202D, 202E of FIG. 2A, or any other rotational displacement of layer 202A from 0 degrees to 360 degrees around a common frame of reference. Further, the one or more layers 304 of tissue scaffold 300 may have greater or fewer number of the plurality of boundary segments 222A and the plurality of crossing segments 224A as provided in layer 202A. Additionally and/or alternatively, region 314 of the tissue scaffold 300 may be similar to any of regions 212A, 212B, 212C, 212D, 212E, or any other region as defined by any possible combination of layers. In some instances, the number of boundary and/or crossing segments of the one or more layers 304 may increase, decrease, or remain constant on a layer-by-layer basis in region 314. For instance, the number of boundary and/or crossing segments of the one or more layers 304 may increase on a gradient from a first number of boundary segments and/or a first number of crossing segments of the first layer of one or more layers 302 of region 312 to a second number of boundary segments and/or a second number of crossing segments of the first layer of one or more layers 306 of region 316.

A porosity, number or pores, and average pore width of the one or more layers 304 of region 314 may transition (e.g., increase or decrease) on a gradient from region 312 of the first porosity having the first plurality of pores, each comprising the first average pore width, to region 316 of a third porosity having a third plurality of pores, each comprising a third average pore width. The transition on the gradient of the porosity, number or pores, and average pore width of the one or more layers 304 of region 314 may occur on a layer-by-layer basis. As such, the one or more layers 304 of region 314 may change incrementally, each having a different porosity, plurality of pores, and/or average pore width. The respective porosities, pluralities of pores, and average pore widths of the one or more layers 304 of region 314 may resemble the nano- and/or micro- structure and/or topography of the transitionary region (e.g., osteochondral region) of native tissue between the first native tissue and the second native tissue.

Region 314 of the tissue scaffold 300 may be formed from a second ratio of the insoluble component to the soluble component of the first material. The second ratio of the soluble component and the insoluble component of the first material may be such that the pitted nano- and/or micro- structure and/or topography resembles that of the transitionary region between the first native tissue and the second native tissue and promotes tissue regeneration in the manner found in the transitionary region. In some cases, the ratio of the soluble component to the insoluble component may transition on a gradient from the first ratio of first region 312 to a third ratio of third region 316. The transition on the gradient of the ratio may occur on a layer-by-layer basis such that each of the one or more layers 304 of region 314 may have a different ratio of the insoluble component to the soluble component.

In some instances, region 314 may have a middle porosity, middle number of pores, and/or middle average pore width in comparison to regions 312 and 316 of tissue scaffold 300. The porosity, number of pores, and average pore width of region 314 may encourage internal fluids and stem cells from an internal bone cavity expelled from a surgical treatment such as microfracture surgery upward and into tissue scaffold 300. Furthermore, the first material used in forming region 314 may have a middle ratio of the insoluble component to the soluble component in comparison to regions 312 and 316.

Region 314 of the arthroscopically implantable three-dimensional tissue scaffold 300 may be configured to extend from a topmost layer of one or more layers 302 of region 312 of the tissue scaffold 300 to a bottommost layer of one or more layers 306 of region 316 of the tissue scaffold 300. In some instances, region 314 may be configured to extend from below or level with a topmost face of the first native tissue to below, above, or level with a bottommost face of the second native tissue.

As stated above, region 316 of the tissue scaffold 300 may be designed to interface with the second native tissue such as cartilage. Each of the one or more layers 306 of region 316 may correspond to a material deposit instance from a rapid prototyping technology at a particular vertical step and/or increment. In some instances, the one or more layers 306 may be formed in a serpentine and/or sinusoidal pattern around a constraining boundary, such as a circle, oval, square, and/or any other regular or irregular geometric shape. In forming the third region 316 of tissue scaffold 300, the serpentine and/or sinusoidal pattern of the one or more third layers 306 may rotate on a layer-by-layer basis. In some cases, each of the one or more layers 306 of region 316 may alternate rotations on a layer-by-layer basis, but may repeat rotations every two layers. As an illustrative and non-limiting example, a first layer of the one or more layers 306 may be of a second serpentine and/or sinusoidal pattern at the first rotation, a second layer of the one or more layers 306 may be of the second serpentine and/or sinusoidal pattern at the second rotation, and then a third layer of the one or more layers 306 may be of the second serpentine and/or sinusoidal pattern and may restart the rotation pattern at the first rotation.

Each of the one or more layers 306 may be rotated from 0 degrees to 360 degrees around a common frame of reference in forming region 316 of tissue scaffold 300. In some instances, the one or more layers 306 of region 316 may be similar to any of layers 202A, 202B, 202C, 202D, 202E of FIG. 2A, or any other rotational displacement of layer 202A from 0 degrees to 360 degrees around a common frame of reference. Further, the one or more layers 306 of tissue scaffold 300 may have greater or fewer number of the plurality of boundary segments 222A and the plurality of crossing segments 224A as provided in layer 202A. In some instances, each of the one or more layers 306 may comprise a quantity of boundary segments such that associated crossing segments are contacting, as depicted in FIG. 3B. In some instances, the number of boundary and/or crossing segments of the one or more layers 306 may increase, decrease, or remain constant on a layer-by-layer basis in region 316. In other instances, each of the layers 306 (e.g., a first layer, a second layer, and so on) of region 316 may have a second number of boundary segments, a second number of crossing segments, and may be of a second serpentine and/or sinusoidal pattern. The second number of boundary segments, second number of crossing segments, and second serpentine and/or sinusoidal pattern of layers 306 may be different than that of the first number of boundary segments, first number of crossing segments, and first serpentine and/or sinusoidal pattern of layers 302.

Additionally and/or alternatively, region 316 of the tissue scaffold 300 may be similar to any of regions 212A, 212B, 212C, 212D, 212E, or any other region as defined by any possible combination of layers. In some instances, the number of boundary and/or crossing segments of the one or more layers 306 may increase, decrease, or remain constant on a layer-by-layer basis in region 316.

Region 316 may be of a third porosity and may have a third plurality of pores, each comprising a third average pore width. In some cases, the third porosity, third plurality of pores, and third average pore width may resemble the nano- and/or microstructure and/or topography of the second native tissue. Region 316 of the tissue scaffold 300 may be formed from a third ratio of the first material comprising at least an insoluble component and a soluble component. In some instances, the third ratio of the soluble component and the insoluble component of the first material may be such that the pitted nano- and/or micro- structure and/or topography resembles that of the second native tissue and promotes tissue regeneration of the second native tissue.

Region 316 of the arthroscopically implantable three-dimensional tissue scaffold 300 may be configured to extend from the topmost face of the surgically induced and/or natural tissue defect to the first native tissue to the topmost face of the second native tissue. For example, in the case of microfracture surgery or other excavatory surgical techniques, region 316 of the tissue scaffold 300 may be configured to be situated beneath, above, or level with the outermost face of the first native tissue and extend beneath, above, or level with the topmost face of the second native tissue surrounding the defect area.

As such, region 316 may have a lesser porosity than that of region 312. For instance, as an illustrative and non-limiting example, a topmost layer of the one or more layers 306 corresponding to the topmost face 307 of scaffold 300 may have a lesser porosity than a bottommost layer of the one or more layers 302 corresponding to the bottommost face 303 of scaffold 300. While the higher porosity at the bottommost layer of the one or more layers 302 corresponding to the bottommost face 303 may invite internal bone fluids and stem cells from an internal bone expelled from a surgical treatment upward and into tissue scaffold 300, the lower porosity at the topmost layer of the one or more layers 306 corresponding to the topmost face 307 of scaffold 300 may hinder and/or prevent the internal fluids and stem cells from exiting tissue scaffold 300.

Due to the nature of rapid prototyping technologies, a bottommost layer of one or more layers 302 of region 312 and a topmost layer of one or more layers 306 of region 316 of tissue scaffold 300 may have unique nano- and/or micro- structure and/or topographies. For reference, discussion is now drawn to FIGS. 5A and 5B, which respectively depict an exemplary nano- and/or micro- scale topographical view 527 of a topmost layer of one or more layers 506 corresponding to a topmost face 507 of an exemplary tissue scaffold 500, and an exemplary nano- and/or micro- scale topographical view 523 of a bottommost layer of one or more layers 502 corresponding to a bottommost face 503 of the exemplary tissue scaffold 500.

Segment 522T/524T, which may be either a boundary segment or crossing segment of the topmost layer of one or more layers 506, is shown in nano- and/or micro-scale topographical view 527. As an illustrative and non-limiting example, scaffold 500 may be manufactured from a rapid prototyping technology, such as fused deposition modeling (e.g., FDM), and the topmost layer of one or more layers 506 may be the first layer deposited during the manufacturing process of scaffold 500, thereby leaving the topmost layer of one or more layers 506 contacting and interfacing with the build tray and/or platform of the FDM device. As shown in exemplary nano- and/or micro- scale topographical view 527 of segment 522T/524T, the topmost layer of one or more layers 506 may be smoother as a result of being deposited directly onto the build tray and/or platform of the FDM device.

The smoothness of the topographical surface of segment 522T/524T may resemble that of native cartilage tissue and thereby prevent catching, snagging, grating, and/or otherwise reduce fricative forces between the topmost layer of one or more layers 506 and adjacent, contacting tissue. Additionally, the smoothness of the topographical surface of segment 522T/524T may encourage stem cell recruitment and regeneration of cartilage tissue. In some cases, the average amplitude between peaks and valleys on the micro scale of the topographical surface of segment 522T/524T may be 1 µm, 5 µm, 10 µm, 15 µm, 20 µm, 25 µm, 30 µm, 35 µm, 40 µm, 45 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, and/or 950 µm. In some instances, the average amplitude between peaks and valleys on the nano scale of the topographical surface of segment 522T/524T may be 1 nm, 5 nm, 10 nm, 15 nm, 20 nm, 25 nm, 30 nm, 35 nm, 40 nm, 45 nm, 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, and/or 950 nm. The amplitude between peaks and valleys of the topographical surface of segment 522T/524T may vary across the surface of segment 522T/524T, but may remain bounded by the ranges provided above wherein no largest amplitude may exceed the range specified.

Similarly, segment 522B/524B, which may be either a boundary segment or crossing segment of a bottommost layer of one or more layers 502, is shown in nano- and/or micro- scale topographical view 523. As an illustrative and non-limiting example, the bottommost layer of the one or more layers 502 may be the last layer deposited during the FDM manufacturing process of scaffold 500, thereby leaving the bottommost layer of one or more layers 502 freely exposed and uncontacted on bottommost face 503. As shown in exemplary nano- and/or micro- scale topographical view 523 of segment 522B/524B, the bottommost layer of one or more layers 502 may be rougher as a result of being the last layer being deposited in the formation of scaffold 500.

The roughness of the topographical surface of segment 522B/524B may resemble that of native bone tissue and thereby encourage stem cell recruitment and regeneration of bone tissue. In some cases, the average amplitude between peaks and valleys on the micro scale of the topographical surface of segment 522B/524B may be 1 µm, 5 µm, 10 µm, 15 µm, 20 µm, 25 µm, 30 µm, 35 µm, 40 µm, 45 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, and/or 950 µm. In some instances, the average amplitude between peaks and valleys on the nano scale of the topographical surface of segment 522B/524B may be 1 nm, 5 nm, 10 nm, 15 nm, 20 nm, 25 nm, 30 nm, 35 nm, 40 nm, 45 nm, 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, and/or 950 nm. In other instances, the average amplitude between peaks and valleys of the topographical surface of segment 522B/524B may be 0.1 mm, 0.2 mm, 0.3 mm, 0.4 mm, 0.5 mm, 0.6 mm, 0.7 mm, 0.8 mm, 0.9 mm, 1.0 mm, 1.1 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, 2.4 mm, 2.5 mm, 2.6 mm, 2.7 mm, 2.8 mm, 2.9 mm, 3.0 mm, 3.1 mm, 3.2 mm, 3.3 mm, 3.4 mm, 3.5 mm, 3.6 mm, 3.7 mm, 3.8 mm, 3.9 mm, 4.0 mm, 4.1 mm, 4.2 mm, 4.3 mm, 4.4 mm, 4.5 mm, 4.6 mm, 4.7 mm, 4.8 mm, 4.9 mm, and/or 5.0 mm.

The amplitude between peaks and valleys of the topographical surface of segment 522B/524B may vary across the surface of segment 522B/524B, but may remain bounded by the ranges provided above wherein no largest amplitude may exceed the range specified.

The first region 312 of tissue scaffold 300 includes a plurality of first layers 302 and at least a first layer of the plurality of first layers 302 is of a first rotational offset from a second layer of the plurality of first layers 302. The third region 316 may sometimes be called second region 316 and may include a plurality of second layers 306. At least a first layer of the plurality of second layers 306 is of a second rotational offset from at least a second layer of the plurality of second layers 306.The first rotational offset of the first layer of the plurality of first layers 302 from the second layer of the plurality of first layers 302 is greater than the second rotational offset of the first layer of the plurality of second layers 306 from the second layer of the plurality of second layers 306.

The plurality of first layers 302 of first region 312 may further include a bottommost layer formed from at least a first boundary segment and at least a first crossing segment. The plurality of second layers 306 of second region 316 may further include a topmost layer formed from at least a second boundary segment and a second crossing segment. In some instances, an exterior surface of at least the first boundary segment and at least the first crossing segment of the bottommost has a first topography comprising a plurality of first peaks and first valleys of a first average amplitude. Further, an exterior surface of at least the second boundary segment and at least the second crossing segment of the topmost layer has a second topography comprising a plurality of second peaks and second valleys of a second average amplitude. In some instances, the first average amplitude is greater than the second average amplitude.

In some cases, at least the first layer of the plurality of first layers 302 may be of a first sinusoidal pattern at a first rotation and at least the second layer of the plurality of first layers 302 may be of the first sinusoidal pattern at a second rotation. At least the first and second layers of the first plurality of layers 302 may be formed from a first number of boundary segments and a first number of crossing segments. Additionally, the first layer of the plurality of second layers 306 may be of a second sinusoidal pattern at the first rotation and at least the second layer of the plurality of second layers 306 may be of the second sinusoidal pattern at a third rotation. In some instances, at least the first and second layers of the second plurality of layers 306 may be formed from a second number of boundary segments and a second number of crossing segments. The first number of boundary segments may be less than the second number of boundary segments and the first number of crossing segments may less than the second number of boundary segments.

The tissue scaffold 300 includes region 314 which may sometimes be called third region 314. Third region 314 is positioned between first region 312 and second region 316 and includes a plurality of third layers 304. At least a first layer of the plurality of third layers 304 is of a first sinusoidal pattern at the first rotation, at least a second layer of the plurality of third layers 304 is of the first sinusoidal pattern at the second rotation, and at least a third layer 304 of the plurality of third layers is of the first sinusoidal pattern at the third rotation.

In some instances, each of the plurality of third layers 304 may include a number of boundary segments and a number of crossing segments. The number of boundary segments and the number of crossing segments may increase on a layer-by-layer basis from a first number of boundary segments and a first number of crossing segments of the first layer of the plurality of third layers 304 to a second number of boundary segments and a second number of crossing segments of the last layer of the plurality of third layers 304. In some cases, the first number of boundary segments and the first number of crossing segments of the first layer of the plurality of third layers 304 may equal to the first number of boundary segments and the first number of crossing segments of the first layer of the plurality of first layers 302. Further, the second number of boundary segments and the second number of crossing segments of the last layer of the plurality of third layers 306 may be equal to the second number of boundary segments and the second number of crossing segments of the first layer of the plurality of second layers 306.

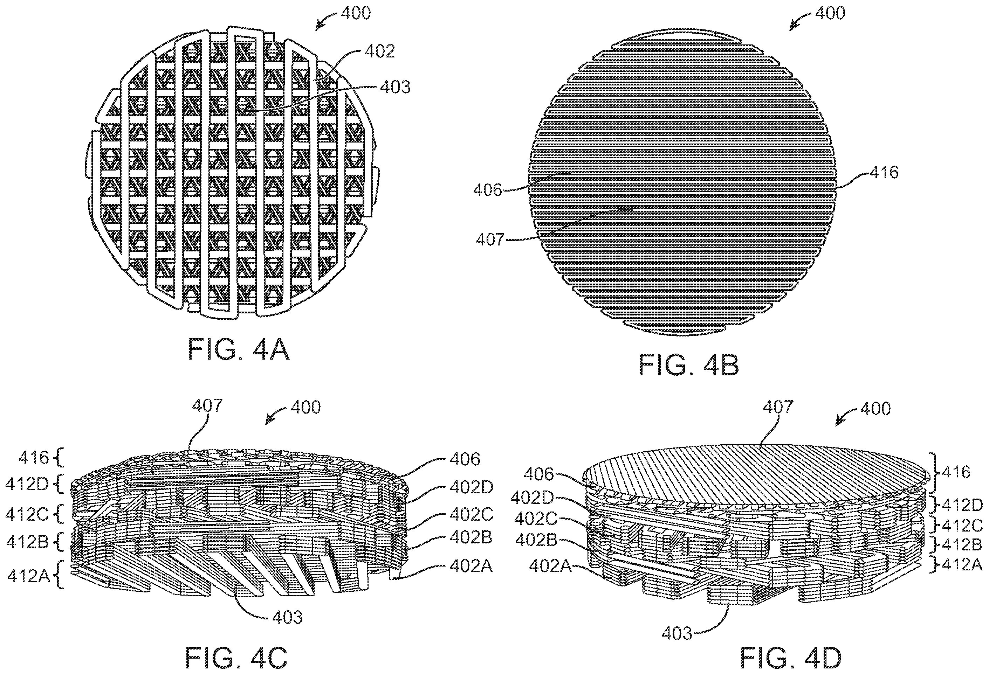

Reference is now made to FIGS. 4A, 4B, 4C, and 4D, which respectively depict a bottom view, a top view, a bottom perspective view, and a top perspective view of a second exemplary tissue scaffold 400 according to one or more embodiments of the disclosure provided herein. The tissue scaffold 400 of FIGS. 4A, 4B, 4C, and 4D is an illustrative and non-limiting example and is exhibited for the purposes of discussing the specificities of the tissue scaffolds contemplated in this disclosure. One of ordinary skill in the art will readily appreciate that the specificities discussed below in regards to tissue scaffold 400 may be incorporated into tissue scaffolds corresponding to defects of any of a plurality of dimensions and corresponding to any of a plurality of bone, cartilage, and/or osteochondral areas. Furthermore, it is to be understood that all disclosures of tissue scaffolds provided herein in regard to embodiments including, but not limited to, porosity, pore number, pore sizing, pore shape, elastic modulus, coatings, pits, pit number, pit sizing, pit shape, material composition, and the like may be applicable to the discussion provided below in regard to the tissue scaffold 400 of FIGS. 4A, 4B, 4C, and 4D. In some instances, tissue scaffold 400 may be designed to facilitate tissue regeneration across one or more types of tissues.

Tissue scaffold 400 may be an arthroscopically implantable three-dimensional tissue scaffold and may be comprised of a plurality of regions. For example, as shown in FIGS. 4C and 4D, tissue scaffold 400 may include a first region 412A, a second region 412B, a third region 412C, a fourth region 412D, and a fifth region 416. Each of regions 412A, 412B, 412C, 412D, and 416 may be formed from one or more layers. For instance, region 412A may be formed from one or more layers 402A (e.g., first layers), region 412B may be formed from one or more layers 402B (e.g., second layers), region 412C may be formed from one or more layers 402C (e.g., third layers), region 412D may be formed from one or more layers 402D (e.g., fourth layers), and region 416 may be formed from one or more layers 406 (e.g., fifth layers). In some instances, tissue scaffold 400 may include a greater number of regions (e.g., three or more regions). Similarly, tissue scaffold 400 may include a fewer number of layers per region or a greater number of layers per region.

Layers 402A, 402B, 402C, 402D, and 406 of regions 412A, 412B, 412C, 412D, and 416 of tissue scaffold 400 may be composed of a first material comprising at least an insoluble component and a soluble component. As an illustrative and non-limiting example, the first material used to fabricate 402A, 402B, 402C, 402D, and 406 of regions 412A, 412B, 412C, 412D, and 416 of tissue scaffold 400 may be a TPU/PVA composite such as Gel-lay or any other material which comprises at least a soluble component and an insoluble component. Alternatively, the first material may consist of a soluble component and an insoluble component such as any of the materials described herein which exhibit the properties of solubility and insolubility, as well as any other such materials.

In some instances, different ratios of the insoluble component to the soluble component of the first material may be used in 402A, 402B, 402C, 402D, and 406. As an illustrative and non-limiting example, one or more first layers 402A may be formed from the first material having a first ratio of the insoluble component to the soluble component, one or more second layers 402B may be formed from the first material having a second ratio of the insoluble component to the soluble component, one or more third layers 402C may be formed from the first material having a third ratio of the insoluble component to the soluble component, one or more fourth layers 402D may be formed from the first material having a fourth ratio of the insoluble component to the soluble component, and one or more fifth layers 406 may be formed from the first material having a fifth ratio of the insoluble component to the soluble component. Accordingly, region 412A may be formed from the first ratio of the first material, region 412B may be formed from the second ratio of the first material, region 412C may be formed from the third ratio of the first material, region 412D may be formed from the fourth ratio of the first material, and region 416 may be formed from the fifth ratio of the first material. Alternatively, layers 402A, 402B, 402C, and 402D, and corresponding regions 412A, 412B, 412C, and 412D may be formed from a first ratio of the first material, and layers 406 corresponding to region 416 may be formed from a second ratio of the first material. In some cases, the ratio of the insoluble component to the soluble component of the first material may change on a layer-by-layer basis within any given region.

Layers 402A, 402B, 402C, 402D, and 406 of regions 412A, 412B, 412C, 412D, and 416 of tissue scaffold 400 may also and/or alternatively be composed of a material (e.g., a second material) other than the first material. The second material used to fabricate layers 402A, 402B, 402C, 402D, and 406 of regions 412A, 412B, 412C, 412D, and 416 may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein. Accordingly, any multitude of materials (e.g., a third material, a fourth material, and so on) may be used to fabricate layers 402A, 402B, 402C, 402D, and 406 of regions 412A, 412B, 412C, 412D, and 416. The materials used, and ratios of components in materials, may change on a region-by-region basis, layer-by-layer basis within regions, and/or any combination thereof.

Each of the one or more layers of layers 402A, 402B, 402C, and 402D of regions 412A, 412B, 412C, and 412D may correspond to a material deposit instance from a rapid prototyping technology at a particular vertical step and/or increment. In some instances, the each of the one or more layers of layers 402A, 402B, 402C, and/or 402D may be formed in a serpentine and/or sinusoidal pattern around a constraining boundary, such as a circle, oval, square, and/or any other regular or irregular geometric shape. In forming the regions 412A, 412B, 412C, and 412D of tissue scaffold 400, the serpentine and/or sinusoidal pattern of layers 402A, 402B, 402C, and 402D may rotate on a region-by-region basis. For example, layers 402A may be of a first serpentine and/or sinusoidal pattern at a first rotation, layers 402B may be of the first serpentine and/or sinusoidal pattern at a second rotation, layers 402C may be of the first serpentine and/or sinusoidal pattern at a third rotation, and layers 402D may be of the first serpentine and/or sinusoidal pattern at a fourth rotation. As such, regions 412A, 412B, 412C, and 412D may be respectively be formed from one or more layers of a similar serpentine and/or sinusoidal pattern, but each of regions 412A, 412B, 412C, and 412D may be at a different rotation relative to a shared frame of reference.

In some instances, regions 412A, 412B, 412C, and 412D of tissue scaffold 400 may be viewed as a single region (e.g., region 412), and layers 402A, 402B, 402C, and 402D may be viewed as sub-groupings of layers within an overarching grouping of layers (e.g., layers 402). Each of the sub-groupings 402A, 402B, 402C, and 402D may be deposited one or more times before proceeding to the next sub-grouping during the manufacture of scaffold 400. For example, sub-grouping 402A may be deposited one or more times at a corresponding one or more vertical steps and/or increments before proceeding to sub-grouping 402B, which may be deposited one or more times at a corresponding one or more vertical steps and/or increments before proceeding to sub-grouping 402C, and so on. In this way, scaffold 400 may be considered to have two regions (e.g., a first region 412 and a second region 416).

A greater or fewer number of sub-groupings of layers 402 may comprise region 412 of scaffold 400. For example, scaffold 400 may include one to three sub-groupings of layers 402 or five or more sub-groupings of layers 402. Regardless of the number of sub-groupings of layers 402, each of the sub-groupings of layers 402 may be at a rotational offset, either unique or repeated, relative to a shared frame of reference. For example, a first sub-grouping of layers may be a first rotation (e.g., 0 degrees), a second sub-grouping of layers may be of a second rotation (e.g., 30 degrees), a third sub-grouping of layers may be of a third rotation (e.g., 60 degrees), and so in. The amount of rotation may any value between 0 degrees and 360 degrees and may be consistent across disparate sub-groupings of layers (e.g., each sub-grouping is rotated at 30 degrees relative to a common frame of reference and a previous sub-grouping) and/or irregular across disparate sub-groupings of layers (e.g., a first sub-grouping may be rotated at 15 degrees relative to a common frame of reference, a second sub grouping may be rotated at 30 degrees relative to the common frame of reference and the first sub-grouping, a third sub-grouping may be rotated at 45 degrees relative to the common frame of reference and the second sub-grouping, and so on). In combination, the sub-groupings may add up to one or more full (e.g., 360 degree) rotations. Alternatively, the sub-groupings may add up to a fractional amount of full rotations (e.g., half a full rotation, one and three quarters full rotations, five and half full rotations, and so on).

Regions 412A, 412B, 412C, and 412D of tissue scaffold 400 may be designed to interface with a first native tissue, such as bone, and may, in combination, be of a first porosity and have a first plurality of pores, each comprising a first average pore width. In some cases, the first porosity, first plurality of pores, and first average pore width may resemble the nano- and/or micro- structure and/or topography of the first native tissue. Regions 412A, 412B, 412C, and 412D of the tissue scaffold 400 may be formed from a first ratio of a first material comprising at least the insoluble component and the soluble component, as described above. When the soluble component is dissolved in a given solvent, the insoluble component may remain and may have an altered nano- and/or micro- structure and/or topography. In some cases, the insoluble component may have a first plurality of pits corresponding to the dissolved soluble component. In some instances, the first ratio of the soluble component and the insoluble component of the first material may be such that the pitted nano- and/or micro- structure and/or topography resembles that of the first native tissue and promotes tissue regeneration of the first native tissue.

Regions 412A, 412B, 412C, and 412D of the arthroscopically implantable three-dimensional tissue scaffold 400 may be configured to be inserted completely below an outermost face of a surgically induced and/or natural tissue defect to the first native tissue. For example, in the case of microfracture surgery or other excavatory surgical techniques (disclosed herein but not forming part of the invention) which cause a pocket or recess to be formed internally and/or inwardly from the outermost face of the first native tissue, regions 412A, 412B, 412C, and 412D of the tissue scaffold 400 may be configured to be situated in the pocket or recess such that a bottommost face 403 of scaffold 400 is contacting a topmost face of the pocket or recess, and a topmost layer of region 412D of the tissue scaffold 400 is below, above, or level with the outermost face of the first native tissue.

Region 416 of the tissue scaffold 400 may be designed to interface with the second native tissue such as cartilage. Each of the one or more layers 406 may be rotated from 0 degrees to 360 degrees around a common frame of reference in forming region 416 of tissue scaffold 400. In some instances, the one or more layers 406 of region 416 may be similar to any of layers 202A, 202B, 202C, 202D, 202E of FIG. 2A, or any other rotational displacement of layer 202A from 0 degrees to 360 degrees around a common frame of reference. Further, the one or more layers 406 of tissue scaffold 400 may have greater or fewer number of the plurality of boundary segments 222A and the plurality of crossing segments 224A as provided in layer 202A. In some instances, each of the one or more layers 406 may comprise a quantity of boundary segments such that associated crossing segments are contacting, as depicted in FIG. 4B. Additionally and/or alternatively, region 416 of the tissue scaffold 400 may be similar to any of regions 212A, 212B, 212C, 212D, 212E, or any other region as defined by any possible combination of layers.

Region 416 may be of a second porosity and may have a second plurality of pores, each comprising a second average pore width. In some cases, the second porosity, second plurality of pores, and second average pore width may resemble the nano- and/or micro- structure and/or topography of the second native tissue. Region 416 of the tissue scaffold 400 may be formed from a second ratio of the first material comprising at least an insoluble component and a soluble component. In some instances, the second ratio of the soluble component and the insoluble component of the first material may be such that the pitted nano- and/or micro- structure and/or topography resembles that of the second native tissue and promotes tissue regeneration of the second native tissue.

Region 416 of the arthroscopically implantable three-dimensional tissue scaffold 400 may be configured to extend from the topmost face of the surgically induced and/or natural tissue defect to the first native tissue to the topmost face of the second native tissue. For example, in the case of microfracture surgery or other excavatory surgical techniques (disclosed herein but not forming part of the invention), region 416 of the tissue scaffold 400 may be configured to be situated beneath, above, or level with the outermost face of the first native tissue and extend beneath, above, or level with the topmost face of the second native tissue surrounding the defect area.

As such, region 416 may have a lesser porosity than that of regions 412A, 412B, 412C, and 412D. For instance, as an illustrative and non-limiting example, a topmost layer of the one or more layers 406 corresponding to the topmost face 407 of scaffold 400 may have a lesser porosity than a bottommost layer of the one or more layers 402A corresponding to the bottommost face 403 of scaffold 400. While the higher porosity at the bottommost layer of the one or more layers 402A corresponding to the bottommost face 403 may invite internal bone fluids and stem cells from an internal bone expelled from a surgical treatment upward and into tissue scaffold 400, the lower porosity at the topmost layer of the one or more layers 406 corresponding to the topmost face 407 of scaffold 400 may hinder and/or prevent the internal fluids and stem cells from exiting tissue scaffold 400.

Disclosed herein, the arthroscopically implantable three-dimensional tissue scaffold may be comprised of a single region, wherein the single region is of a first material including at least an insoluble component and a soluble component. The single region may be comprised of a plurality of layers and may be fabricated in a gradient such that a bottommost layer of the arthroscopically implantable three-dimensional tissue scaffold may be of a first porosity and have a first plurality of pores, each comprising a first average pore width, and that a topmost layer of the tissue scaffold may be of a second porosity and have a second plurality of pores, each comprising a second average pore width. Between the bottommost layer and the topmost layer of the tissue scaffold, the porosity, number of pores, and/or pore width may transition (e.g., increase or decrease) on a gradient on a layer-by-layer basis from the first porosity having the first plurality of pores, each comprising the first average pore width, to the second porosity having the second plurality of pores, each comprising the second average pore width. Non-limiting examples of types of gradients may include linear, exponential, logarithmic, radial, reflected, and the like. Accordingly, the term gradient may be used to describe differences in one or more of the porosity, number of pores, and average pore with on a layer-by-layer basis.

Additionally and/or alternatively, the first material comprising at least the insoluble component and the soluble component may vary in ratio of the insoluble component to the soluble component along the gradient. For example, the bottommost layer of the arthroscopically implantable three-dimensional tissue scaffold may have a first ratio of the soluble component to the insoluble component, and the topmost layer of the tissue scaffold may have a second ratio of the soluble component to the insoluble component. Between the bottommost layer and the topmost layer of the tissue scaffold, the ratio of the soluble component to the insoluble component of the first material may transition (e.g., increase or decrease) on the gradient from the first ratio to the second ratio. The gradient on which the ratio transitions may be of the same or different type than the gradient described above in regard to the porosity, number of pores, and pore widths.

In such instances, the topmost layer, bottommost layer, and any layers there between may be of a first porosity and have a first plurality of pores, each comprising a first average pore width. Additionally, the ratio of the insoluble component to the soluble component may be of a first ratio and be consistent across the tissue scaffold.

### Methods of Manufacturing Arthroscopically Implantable Tissue Scaffolds

Scaffolds as disclosed herein may be manufactured using a printing method. Scaffolds may be manufactured using a rapid prototyping technology method. Scaffolds may be manufactured using an extrusion method. Scaffolds may be manufactured using an electrospinning method. One or more manufacturing methods as described herein may be utilized alone or in combination to produce a scaffold. Methods may manipulate polymeric materials, metallic materials, native scaffolding materials (such as collagen, fibronectin), or any combination thereof. Different regions or different layers of a scaffold may be manufacturing using different manufacturing parameters or different methods.

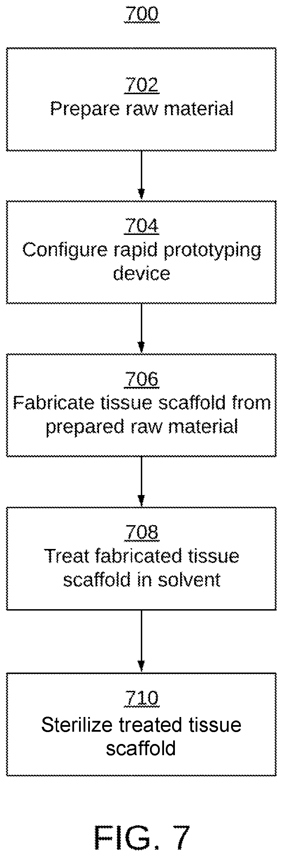

Reference is now made to FIG. 7, which depicts a non-limiting flow diagram illustrating an exemplary method 700 of fabricating a tissue scaffold disclosed herein, but not claimed.

At step 702, raw material for manufacturing a tissue scaffold may be prepared. In the case of manufacturing the tissue scaffold by a rapid prototyping technology such as fused deposition modeling (e.g., FDM), as disclosed herein but not claimed, a filament-based approach may be used. In some instances, a filament may be composed of a first material having at least an insoluble component and a soluble component. For example, the filament may be a TPU/PVA composite such as Gel-lay or any other material which comprises at least a soluble component and an insoluble component. Alternatively, the filament may consist of a soluble component and an insoluble component such as any of the materials described herein which exhibit the properties of solubility and insolubility, as well as any other such materials. In other instances, the filament may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein. In manufacturing the tissue scaffold with a multi-extrusion FDM device, a plurality of filaments may be used corresponding to the material composition of the respective layers and/or regions of the tissue scaffold.

In some cases, the filament may be composed of one or more sub-sections, wherein each of the one or more sub-sections correspond to a particular material used in fabricating layers and/or regions of a tissue scaffold. For example, in the event that the tissue scaffold has three regions, each of which having a different ratio of the insoluble component to the soluble component of the first material, then the filament may be composed of three sub-sections, wherein a first sub-section has a first ratio of the insoluble component to the soluble component, the second sub-section has a second ratio of the insoluble component to the soluble component, and a third sub-section has a third ratio of the insoluble component to the soluble component. In some cases, the one or more sub-sections of the filament may correspond to different materials. For example, in the event that the tissue scaffold has two regions, the first region composed from a first material and the second region being composed from a second material, then the filament may be composed of two sub-sections, wherein a first sub-section is made of the first material and the second sub-section is made of the second material. In other cases, the filament may be composed on a gradient of the first material comprising the insoluble component and the soluble component, wherein the gradient corresponds to an increase and/or decrease of the ratio of the insoluble component to the soluble component. By using a single filament with different sub-sections, manufacture of the tissue scaffold may occur on an FDM device with a single printhead without requiring changing of filament during the manufacturing process.

Additionally and/or alternatively, in the case of manufacturing the tissue scaffold by a rapid prototyping technology such as selective laser sintering (SLS) and/or selective laser melting (SLM), as disclosed herein but not claimed, a powder-based approach may be used. In some instances, a powder may be composed of a first material having at least an insoluble component and a soluble component. For example, the powder may be a TPU/PVA composite such as Gel-lay or any other material which comprises at least a soluble component and an insoluble component. Alternatively, the powder may consist of a soluble component and an insoluble component such as any of the materials described herein which exhibit the properties of solubility and insolubility, as well as any other such materials. In other instances, the powder may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein.

In some cases, the powder may be a substrate composed of one or more sub-sections, wherein each of the one or more sub-sections correspond to a particular material used in fabricating layers and/or regions of a tissue scaffold. For example, in the event that the tissue scaffold has three regions, each of which having a different ratio of the insoluble component to the soluble component of the first material, then the powder substrate may be composed of three sub-sections, wherein a first sub-section has a first ratio of the insoluble component to the soluble component, the second sub-section has a second ratio of the insoluble component to the soluble component, and a third sub-section has a third ratio of the insoluble component to the soluble component. In some cases, the one or more sub-sections of the powder substrate may correspond to different materials. For example, in the event that the tissue scaffold has two regions, the first region composed from a first material and the second region being composed from a second material, then the powder substrate may be composed of two sub-sections, wherein a first sub-section is made of the first material and the second sub-section is made of the second material. In other cases, the powder substrate may be composed on a gradient of the first material comprising the insoluble component and the soluble component, wherein the gradient corresponds to an increase and/or decrease of the ratio of the insoluble component to the soluble component. By using a powder substrate with different sub-sections, manufacture of the tissue scaffold may occur on an SLS and/or SLM device without having to change material powder during the manufacturing process.

Additionally and/or alternatively, in the case of manufacturing the tissue scaffold by a rapid prototyping technology such as stereolithography (e.g., SLA), as disclosed herein but not claimed, a resin-based approach may be used. In some instances, a resin may be composed of a first material having at least an insoluble component and a soluble component. For example, the resin may be a TPU/PVA composite such as Gel-lay or any other material which comprises at least a soluble component and an insoluble component. Alternatively, the resin may consist of a soluble component and an insoluble component such as any of the materials described herein which exhibit the properties of solubility and insolubility, as well as any other such materials. In other instances, the resin may be composed of one or more of polylactic acid (PLA), poly-L-lactic acid (PLLA), polyglycolic acid (PLGA), polycaprolactone (PCL), polydioxanone (PDO), collagen, fibrin, and the like, as well as any of the other materials described herein.

In some cases, the resin may be composed of one or more sub-sections, wherein each of the one or more sub-sections correspond to a particular material used in fabricating layers and/or regions of a tissue scaffold. For example, in the event that the tissue scaffold has three regions, each of which having a different ratio of the insoluble component to the soluble component of the first material, then the resin may be composed of three sub-sections, wherein a first sub-section has a first ratio of the insoluble component to the soluble component, the second sub-section has a second ratio of the insoluble component to the soluble component, and a third sub-section has a third ratio of the insoluble component to the soluble component. In some cases, the one or more sub-sections of the resin may correspond to different materials. For example, in the event that the tissue scaffold has two regions, the first region composed from a first material and the second region being composed from a second material, then the resin may be composed of two sub-sections, wherein a first sub-section is made of the first material and the second sub-section is made of the second material. In other cases, the resin may be composed on a gradient of the first material comprising the insoluble component and the soluble component, wherein the gradient corresponds to an increase and/or decrease of the ratio of the insoluble component to the soluble component. By using a resin with different sub-sections, manufacture of the tissue scaffold may occur on an SLA device without having to change material resin during the manufacturing process.

At step 704, the rapid prototyping device to be used in the manufacturing of the tissue scaffold may be configured. In some cases, specific parameters associated with the rapid prototyping device may be set in order to produce a tissue scaffold according to the specifications provided here. For example, in regard to printing via an FDM device, extrusion temperature may be set to 250 degrees Celsius, environmental humidity may be maintained at less than 30%, minimum layer time (e.g., time between depositing successive layers) may be set to 5 seconds, and print speeds on the x-axis, y-axis, and/or z-axis may be set to greater than 10 mm/sec. In some cases, however, extrusion temperature may be set to greater than or less than 250 degrees Celsius, environmental humidity may be maintained at greater than 30%, minimum layer time (e.g., time between depositing successive layers) may be set to less than or greater than 5 seconds and print speeds on the x-axis, y-axis, and/or z-axis may be set to less than 10 mm/sec. In some instances, layer thickness (e.g., z-axis height on a layer-by-layer basis) may be set to 250 µm. In other instances, layer thickness may be set to greater than or less than 250 µm. Additionally, nozzles on the FDM device through which the filament is extruded may range between 0.1 mm and 1 mm in diameter. In some instances, however, the nozzles may be of a greater or smaller diameter. The nozzle diameter may affect a diameter of the filament as extruded after being heated to an extrudable temperature.

For other rapid prototyping technologies such as SLA and SLS, the active material component may have a consistent particle size as comprised within the greater powder or resin composition. In some cases, the consistent particle size may be between 50 µm and 60 µm, but may also be on a larger or smaller scale. Furthermore, in either the case of SLA or SLS, the temperature at the contact surface between the powder or resin composition and the laser emission from the rapid prototyping device may be less than 250 degrees Celsius. In some cases, the temperature may be greater than 250 degrees Celsius.

At step 706, the tissue scaffold may be fabricated by the rapid prototyping device configured at step 704 from the raw material prepared at step 702. In some instances, a single tissue scaffold may be manufactured in one print instance. In other instances, a plurality of tissue scaffolds may be manufactured in a one print instance. In such instances, the raw material prepared at step 702 may accommodate the printing of the plurality of scaffolds in one print instance.

At step 708, the tissue scaffold fabricated at step 706 may be treated with in a solvent. For example, the tissue scaffold may be exposed to, submerged under, and/or contacted with a solvent. As stated above, solvents may include, without limitation, water (H2O), acetic acid, acetone, acetonitrile, benzene, 1-butanol, 2-butanol, 2-butanone, t-butyl alcohol, carbon tetrachloride, chlorobenzene, chloroform, cyclohexane, 1,2-dichloroethane, diethylene glycol, diethyl ether, diglyme (diethylene glycol dimethyl ether), 1,2-dimethoxy-ethane (glyme, DME), dimethyl-formamide (DMF), dimethyl sulfoxide (DMSO), 1,4-dioxane, ethanol, ethyl acetate, ethylene glycol, glycerin, heptane, hexamethylphosphoramide (HMPA), hexamethylphosphorous triamide (HMPT), hexane, methanol, methyl t-butyl ether (MTBE), methylene chloride, N-methyl-2-pyrrolidinone (NMP), nitromethane, pentane, petroleum ether (ligroin), 1-propanol, 2-propanol, pyridine, tetrahydrofuran (THF), toluene, triethyl amine, o-xylene, m-xylene, p-xylene, and D-limonene.

In some instances, in order to accelerate the dissolving of the soluble component from the soluble component, the tissue scaffold may be shaken, agitated, moved, vibrated, and/or otherwise mechanically displaced within the solvent. Additionally and/or alternatively, a tissue scaffold composed of the material with the soluble and insoluble components may be fastened, attached, and/or otherwise fixed and the solvent may be shaken, agitated, moved, vibrated, and/or otherwise mechanically displaced around, over, and/or through the tissue scaffold.

At step 710, the tissue scaffold fabricated at step 706 and treated at step 708, may be sterilized. In some instances, the sterilization process may include one or more of physical, chemical, steam, dry heat, ethylene oxide, and/or radiation sterilization processing.

In some cases, the method of manufacturing the three-dimensional scaffold may include fabricating a first region by printing a first plurality of layers, wherein at least a first layer of the plurality of first layers is of a first rotational offset from at least a second layer of the plurality of first layers, and fabricating a second region by printing a second plurality of layers, wherein at least a first layer of the plurality of second layers is of a second rotational offset from at least a second layer of the plurality of second layers. In some instances, the first rotational offset may be greater than the second rotational offset.

In some cases, the plurality of first layers of the first region may further include a bottommost layer formed from at least a first boundary segment and at least a first crossing segment, and the plurality of second layers of the second region may further include a topmost layer formed from at least a second boundary segment and a second crossing segment. Furthermore, an exterior surface of at least the first boundary segment and at least the first crossing segment of the bottommost may have a first topography comprising a plurality of first peaks and first valleys of a first average amplitude and an exterior surface of at least the second boundary segment and at least the second crossing segment of the topmost layer may have a second topography comprising a plurality of second peaks and second valleys of a second average amplitude. In some cases, the first average amplitude may be greater than the second average amplitude.

In some instances, at least the first and second layers of the first plurality of layers may be formed from a first number of boundary segments and a first number of crossing segments. Further, at least the first and second layers of the second plurality of layers may be formed from a second number of boundary segments and a second number of crossing segments. In some cases, the first number of boundary segments may be less than the second number of boundary segments and the first number of crossing segments may be less than the second number of boundary segments. Additionally, the three-dimensional scaffold may be printed from a from a first material having at least a soluble component and an insoluble component and the soluble component of the first material may be soluble in water.

### Methods of Arthroscopically Implanting Tissue Scaffolds

Disclosed herein, but not forming part of the invention, is the implantation of one or more scaffolds into a subject, such as a subject in need thereof. Disclosed herein, but not forming part of the invention, is the implantation of a scaffold via a surgical method. Disclosed herein, but not forming part of the invention, is the implantation of a scaffold via an arthroscopic method. Disclosed herein, but not forming part of the invention, is the implantation of a scaffold via a minimally invasive method. Disclosed herein, but not forming part of the invention, is the implantation of a scaffold which may be compacted, folded, or rolled, during delivery and expanded, unfolded, or unrolled upon arrival to the site of delivery.

The tissue scaffolds disclosed herein may be used to treat large surface cartilage injuries, which may include, for example, osteoarthritis, sports injuries, traumatic injuries, or any damage that is otherwise sustained by the surface cartilage, as well as any damage to the underlying junction between that cartilage and subchondral bone. These types of injuries may have the thickness of cartilage (e.g., approximately 2 mm) and a large comparative area (e.g., approximately 1 cm to 2.5 cm in diameter). These injuries are currently treated by therapies that include, for example, the removal of damaged tissue and replacement with an implant or cadaver tissue. Such procedures require the entire joint space to be opened in what is called an arthrotomy, so that significant portions of cartilage and bone can be removed, and a large implant or graft can be properly placed in the joint space. Currently, graft tissue and other treatments of this size cannot be implanted arthroscopically, which results in patients having to undergo a more invasive procedure with longer recovery times.

Disclosed herein, but not forming part of the invention, are tissue scaffolds that may be implanted arthroscopically. In some cases, a tissue scaffold of the disclosure may have outer dimensions similar to those described above to treat a large surface cartilage injury. In some cases, a tissue scaffold of the disclosure may have a thickness of about 1.5 mm to about 2 mm. In some cases, a tissue scaffold of the disclosure may have a thickness of greater than about 2 mm. In some cases, tissue scaffolds of the disclosure may have a diameter of about 1 cm to about 2.5 cm. In some cases, a tissue scaffold of the disclosure may have a diameter of greater than about 2.5 cm. In some cases, a tissue scaffold of the disclosure may have a diameter of less than about 4 cm. In some cases, a tissue scaffold of the disclosure may be highly flexible and/or durable (see, e.g., FIG. 6). The disclosed tissue scaffold may include a combination of 3D printed porous structures and a unique elastic and nanoporous material, as described herein. Disclosed herein, the disclosed tissue scaffold may be fit into a joint space arthroscopically. For example, the disclosed tissue scaffold may be rolled up or otherwise deformed, so that the scaffold may fit down the working channel of an arthroscope or a secondary arthroscopic tool (e.g., 1-4 mm). Once the disclosed tissue scaffold is introduced to the joint space, the scaffold can be unrolled and installed using any existing surgical techniques (e.g., chondral darts or pins, fibrin glue, surgical adhesives or suture anchors).

### REFERENCE EXAMPLE 1 - IMPLANTATION OF A SCAFFOLD

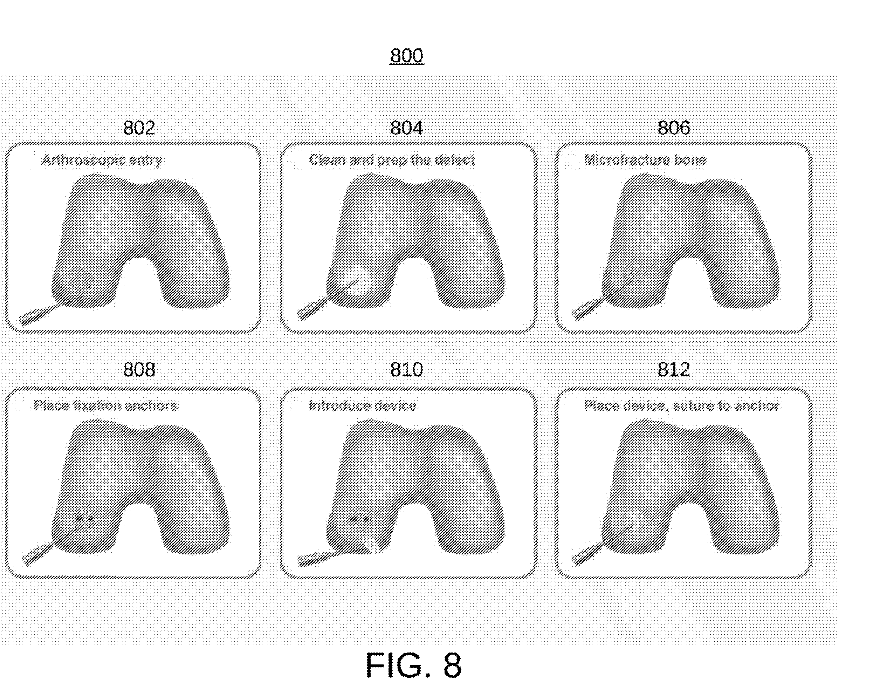

Reference is now made to FIG. 8, which depicts a non-limiting example of a procedure 800 for implanting a tissue engineering scaffold arthroscopically into an osteochondral defect.

At step 802, an arthroscopic entry may be made into the tissue defect area. In some instances, the tissue defect area may comprise a bone and/or cartilage defect to the knee. In other instances, the defect area may be a defect to any osteochondral interface in the body.

At step 804, the defect area may be cleaned and prepped for application of surgical techniques, as well as a tissue scaffold as described herein. Specifically, in the event that the tissue defect includes damage to cartilage at the site of arthroscopic entry, the cartilage area in and around the defect area may be cleaned, thereby exposing the underlying bone surface.

At step 806, a microfracture surgical technique may be applied to the defect area cleaned and prepped at step 804. In some cases, a plurality of punctures and/or fractures may be made to the outer layer of bone, thereby exposing and/or creating channels to the interior bone cavity. Alternatively, other excavatory surgical techniques may be applied which cause a pocket and/or recess to be formed inwardly from the outermost bone layer. In some instances, one or more channels may be drilled into the bone in order to access the internal bone cavity.

At step 808, fixation anchors may be placed at the microfracture and/or excavatory surgical site. The surgical anchors may be used to help attach the tissue scaffold to the defect sight. In some instances, however, no surgical anchors may be used, and instead the scaffold may be attached to the defect area in the manor described at step 812.

At step 810, the tissue scaffold may be introduced into the defect area. In some cases, the disclosed tissue scaffold may be fit into a joint space arthroscopically. For example, as stated above, in forming the tissue scaffold, each of the layers comprising the various regions of the scaffold may be rotated at acute angles (e.g., less than 90 degrees) on a layer-by-layer basis. The rotation of layers may further occur across all regions of the tissue scaffold such that the rotational offset between each layer of the tissue scaffold is at an acute angle. The angle of rotation between each of the layers of the scaffold may be acute, but may vary on a layer-by-layer basis. By doing so, the resulting tissue scaffold may display increased foldability, deformability, and/or otherwise the rotational pliability along an axis parallel to a front face of the resulting tissue scaffold. The resulting mechanical properties may enable the scaffold to be rolled along the axis parallel to the front face of the scaffold such that the scaffold, when rolled, may be able to be fitted into an arthroscopic tool for surgical insertion into the tissue defect area.

As such, the disclosed tissue scaffold may be rolled up or otherwise deformed, so that the scaffold may fit down the working channel of an arthroscope or a secondary arthroscopic tool in order to be entered into the defect area. In some instances, the arthroscopic tool may be a catheter and the scaffold may be deformed in order to fit inside an interior portion of the catheter. After the catheter is arthroscopically inserted into the defect site, an outer sheath on the catheter may be pulled outwardly thereby releasing and undeforming the scaffold into the tissue defect area.

In some cases, a tissue defect or portion thereof (such as a knee cavity) may be treated in order to create a dry operating environment (substantially free of moisture) within the tissue defect, adjacent thereto the tissue defect, or a combination thereof. For example, the tissue defect may be treated by gas insufflation in order to create a dry operating environment within an interior portion of the tissue defect (such as a knee joint). In some cases, the gas utilized for the gas insufflation may comprise a substantially pure gas, such as carbon dioxide. In some cases, the gas may comprise a mixture of gases. In some cases, the at least a portion of the gas used in the gas insufflation may be carbon dioxide (e.g., CO2). The process of drying or removing moisture from an operating environment may be performed (i) before administration of a scaffold, (ii) after administration of a scaffold, (iii) during administration of a scaffold, duration a microfracture technique or during another excavatory surgical technique, or (iv) any combination thereof. Through gas insufflation, the tissue (such as a joint) may be fixed at a particular pressure level through continuous administration of a gas into the tissue defect (such as a joint cavity).

At step 812, the tissue scaffold may be placed into the tissue defect area and sutured to the fixation anchors inserted into the defect area at step 808. In some cases, the implant may be secured into the injury site via a variety of surgical methods, including suture anchors, biodegradable pins, staples, sutures or surgical adhesives such as fibrin glue, or other types of crosslinking adhesive materials that are biocompatible and biodegradable. In some cases, the gas insufflation of the tissue defect (such as a knee joint) may create a drier environment or an environment having substantially less moisture as compared to a tissue defect without gas insufflation such that an adhesive advantageously adheres a portion of the scaffold better to the surrounding tissue (such as bone tissue, cartilage tissue, or a combination thereof).

Disclosed herein, but not forming part of the invention, is a method of treating a subject having a tissue defect comprising surgically implanting a three-dimensional tissue scaffold into the tissue defect of the subject, thereby treating the subject, wherein the three-dimensional tissue scaffold may comprise a first region including a plurality of first layers, wherein at least a first layer of the plurality of first layers may be of a first rotational offset from at least a second layer of the plurality of first layers, a second region including a plurality of second layers, wherein at least a first layer of the plurality of second layers may be of a second rotational offset from at least a second layer of the plurality of second layers, wherein the first rotational offset may be greater than the second rotational offset.

Because the disclosed tissue scaffolds may be applied arthroscopically, the scaffold provides numerous benefits over existing therapies. For example, an arthroscopic procedure is faster and disturbs far less healthy tissue. As a result, patients may have reduced pain, may have a shorter recovery, and may have better long-term outcomes.

### EXAMPLE 1 - IN VITRO AND MECHANICAL STUDIES

*In vitro* studies were carried out to characterize the response of mesenchymal stem cells (e.g., MSCs) in relation to a 3D printed nanoporous thermoplastic polyurethane (e.g., nTPU) tissue engineering scaffold. Previous studies *in vitro* and *in vivo* suggested that the 3D printed nanoporous nTPU scaffold was osteogenic and promoted vascularization, both of which are required for healthy attachment to bone in a clinical setting. Thus, additional *in vitro* testing focused on confirming and expanding on this observed effect on an implant designed to replace lost or damaged cartilage on the articulating surface of the knee. In preparation for further *in vitro* testing, 3D porous bi-phasic disks were designed, 3D printed, sterilized, and subsequently evaluated via MSC cell study, assays, confocal imaging, and quantitative analysis. Additionally, mechanical testing studies were performed, including both compression and fatigue tests, and SEM images of the fatigued samples were taken and analyzed.

The goal of the *in vitro* study was to show that when nTPU was printed into a bi-phasic porous disk, the nTPU material may maintain biocompatibility and osteoinconductivity with regards to MSC growth. An additional goal was to demonstrate that, when co-cultured with MSCs and endothelial cells, the implants may form vascularized bone. Vascularization is critical to forming a lasting and effective bond between bone and implant. MSC growth and development was evaluated via picogreen cell counting assay, alkaline phosphatase, and calcium deposition. Vascular growth was evaluated using confocal microscopy images of co-cultured cells, which were then processed using an image processing software. This was done to quantify the observed growth of vascular structures and to compare the quantification to a culture with MSCs alone.

In the *in vitro* study, the 3D printed nanoporous nTPU implants showed both the support of MSC growth and greater bone-differentiation markers than a control group. When co-cultured, the 3D printed nanoporous nTPU implants also showed a denser and better developed vascular network.

The mechanical portion of the study was conducted in two phases. Phase I employed axial compression to compare the Young's moduli of 3D printed samples when the samples were wet and dry. An important feature of the 3D printed nanoporous nTPU scaffold may be the devices ability to absorb water, and take on cartilage-like mechanical properties. In phase II, additional cycle testing was conducted to show that the wet samples may endure repeated loading without significant physical deformation or changes in mechanical properties.

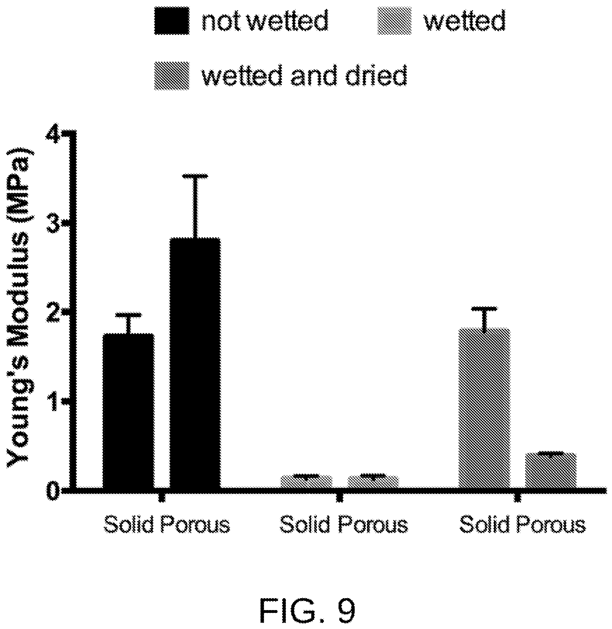

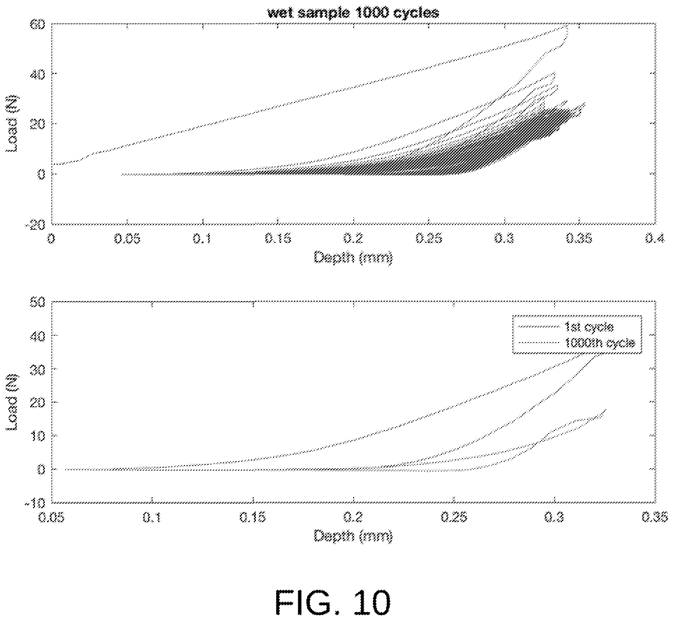

Mechanical testing demonstrated that the material, when wetted, has a cartilage-like Young's modulus and that after 1000 cycles there is no significant deterioration of mechanical properties or observed deformation. For example, as shown in FIG. 9, the wetted 3D printed nanoporous nTPU implants exhibited a Young's modulus similar to that of native cartilage tissue (e.g., in the range of 0.5 to 0.9 MPa). Additionally, as shown in FIG. 10, the 3D printed nanoporous nTPU implants demonstrated comparable depth displacement throughout cycle load testing, and specifically in relation to the first cycle and the 1000^{th} cycle.

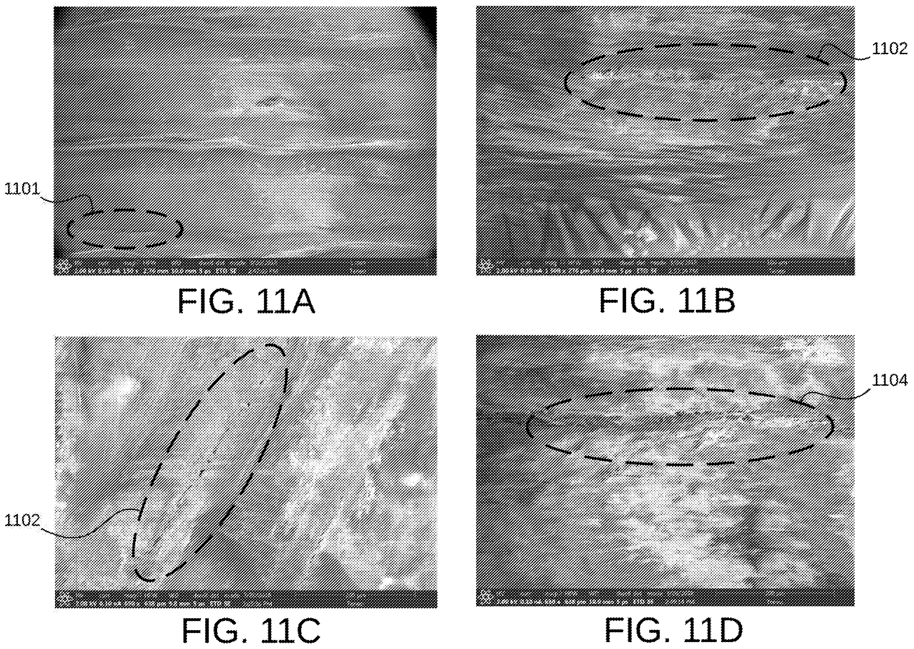

FIGS. 11A, 11B, 11C, and 11D depict scanning electron microscopy (e.g., SEM) images of a 3D printed nanoporous nTPU implant after cycle loading. In FIG. 11A, which shows a low magnification image of the surface of the 3D printed nanoporous nTPU implant after cycle loading, a small stress crack is observed at area 1101 near the edge of the implant. In FIG. 11B, a high resolution image of the stress crack is provided and particularly denoted by area 1102. FIGS 11C and 11D respectively show, at areas 1103 and 1104, additional magnified images of the small stress crack. As demonstrated by FIGS. 11A-11D, only minimal deformation and stress crack formation was observed, and only at the edge of the 3D printed nanoporous nTPU implant.

### EXAMPLE 2 - MANUFACTURING ANALYSIS

During manufacturing analysis, the printability of nTPU was assessed across a two-phase approach. In the first phase, the first step was to perform small scale lab tests which were followed by an assessment and decision to go forward to phase two with actual print tests.

By choosing specific test artifacts, the various barriers between a new material and processability via various rapid prototyping methods were assessed. Three different categories of artifacts or parts were produced: parameter setting, part/design properties, and actual geometries.

The manufacturing feasibility conducted during manufacturing analysis had several objectives. First, to evaluate the printability of nTPU across various rapid prototyping methods. For example, in regard to the rapid prototyping method of selective laser sintering (e.g., SLS), small scale labs tests that the material had to clear were SSC analysis to determine a thermal printing window, TGA analysis to show material weight loss, analysis of nTPU powder to determine particle size distribution, dynamic angle of repose analysis to predict powder flow properties, a powder spreading experiment to determine hypothetical layer uniformity during pre-printing deposition, and a series of rheological measurements of the powder and melted material. Furthermore, in regard to FDM, various test prints were conducted in order to optimize FDM as a manufacturing process. Print effectiveness was measured by comparing non-dried and dried filament material used to create "temperature towers," print speed "rings," layer time "pyramids," an overhang angle part, tensile testing dog bones, and a prototype of the 3D printed nanoporous nTPU implant.

Based on the results, it was determined that FDM was the ideal rapid prototyping method for nTPU. In the FDM based approach for phase two, the filament processed well on a commercial FDM device and seems to have a broad processing window in both speed as well as nozzle temperature. While drying is a necessary and important pretreatment step, no other recommendations are prescribed in preparing the material for printing.

The flatwise printed tensile bars (e.g., dog bones) exhibited mechanical properties of high stiffness with a low elongation. Depending on the application and required orientation, there might be a need in the future to check the mechanical properties either in other modes of loading (e.g., flexure or torsion) or in other part orientations (e.g., edge or upright). With respect to actual part geometries, the nTPU filament is able to be processed properly into the desired shapes.

### EXAMPLE 3 - ANIMAL STUDY

The animal study involved the evaluation of 3D printed nanoporous nTPU implants in large animals. Both 3D printed, sterilized nanoporous nTPU devices and instructions for implantation were provided to a third-party animal research entity, which then used the devices and implantation procedures to treat cartilage defects in the knees of goats.

The study employed six skeletally mature female Spanish goats weighing about 100 pounds (e.g., lbs) at study start. The 3D printed nanoporous nTPU implant devices that were tested are intended for cartilage implantation on the articulate surface of the knee in humans. The 3D printed nTPU implant devices are intended to repair so called "focal cartilage defects" which are more than 1 cm in diameter in the condyles of the knee, and return a person to full mobility over a period of four months.

The purpose of the large animal study was to investigate the toxicity, tissue compatibility, and foreign body reactions of the test device when used to repair a full-thickness cartilage defect, and to compare the treatment results of the device with corresponding treatment results of microfracture surgery, the current standard of treatment. A previous study, which involved the implantation of a single device in an osteochondral defect in the knee of a rodent, demonstrated safety over periods out to three months post-implantation.

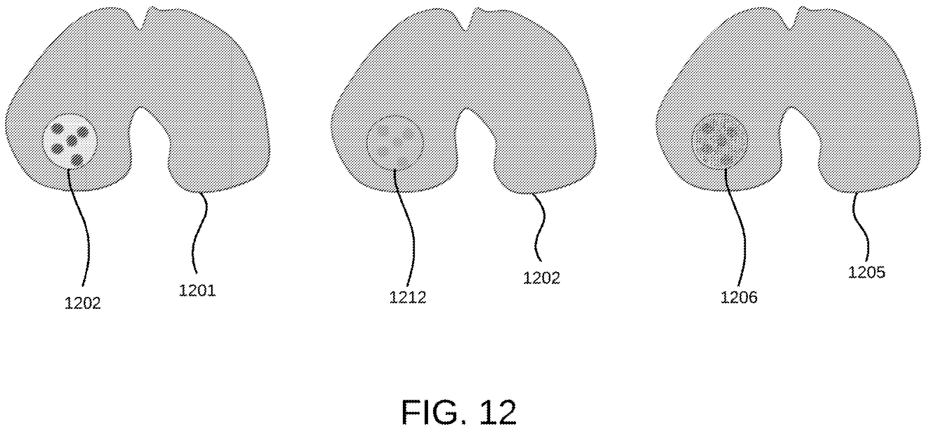

In the large animal study, the 3D printed nanoporous nTPU device was implanted in full-thickness cartilage defects in the right and left femoral condyles. Goats received a surgically induced full-thickness defect in both knees followed by microfracture treatment and implantation of either a solid nTPU device or a 3D printed nanoporous nTPU device in one knee, while the other knee was given a full-thickness cartilage defect with microfracture treatment alone. As a non-limiting, illustrative example, FIG. 12 shows femoral condyle cross-section 1201 associated with microfracture treatment 1202, femoral condyle cross-section 1203 associated with solid nTPU device treatment 1212, and femoral condyle cross-section 1205 associated with 3D printed nanoporous nTPU device treatment 1206.

Once the study was concluded (e.g., after four months) samples from all surgical sites were removed and evaluated. Histological studies were conducted in addition to biomechanical testing.

The large animal study was intended to show that the 3D printed nanoporous nTPU implant may perform in a clinical setting to treat large cartilage injuries to the articulating surface of the knee. Specific objectives of the large animal study were to evaluate recovery time after surgery, long term clinical recovery at four months, compatibility with existing surgical techniques, tooling, and fixation, overall quality of the implant and observed healing as compared to microfracture treatment and a non-3D printed implant, and the quality and specificity of the new tissue formed. The last objective was evaluated histologically, and sought to compare the types of tissue formed (e.g., fibrocartilage versus actual bone and new cartilage) in microfracture treatment as compared to the 3D printed nanoporous nTPU implant.

The animal study revealed significant results with respect to the stated objectives. First, the initial implantation of the device demonstrated that the geometry of the 3D printed nanoporous nTPU implant was highly flexible, and may be formed into place by the surgeon. Thus, the study showed that the 3D printed nanoporous nTPU implant does not need to be fully custom made. The study further showed that the 3D printed nanoporous nTPU implant may be installed over the microfractured bone successfully using chondral darts, biodegradable pins that are widely used to fix graft tissue in place. After 24 hours all the goats that received the 3D printed nanoporous nTPU implant recovered and were walking. This is significant as existing treatments require patients to apply absolutely no weight to their knee for the first six weeks. After the four month study had concluded, no animals contracted infections. Finally, at the four month endpoint, it was observed that the 3D printed nanoporous nTPU implant was most successful.

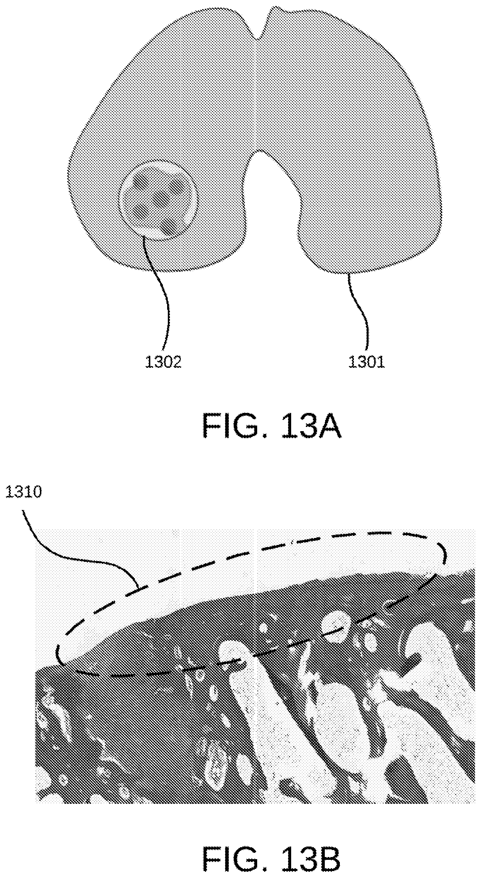

First, the microfracture treatment area showed little to no visible tissue growth and, in some cases, indicated deterioration of healthy tissue. As a non-limiting, illustrative example, FIG. 13A shows femoral condyle cross-section 1301 associated with microfracture treatment area 1302 at the conclusion of the four-month period with minimal healing and some new damage. Furthermore, as shown in FIG. 13B, which depicts a hematoxylin and eosin (e.g., H&E) histological stain of the microfracture treatment area after the four-month period of time, area 1310 shows little to no new cell formation.

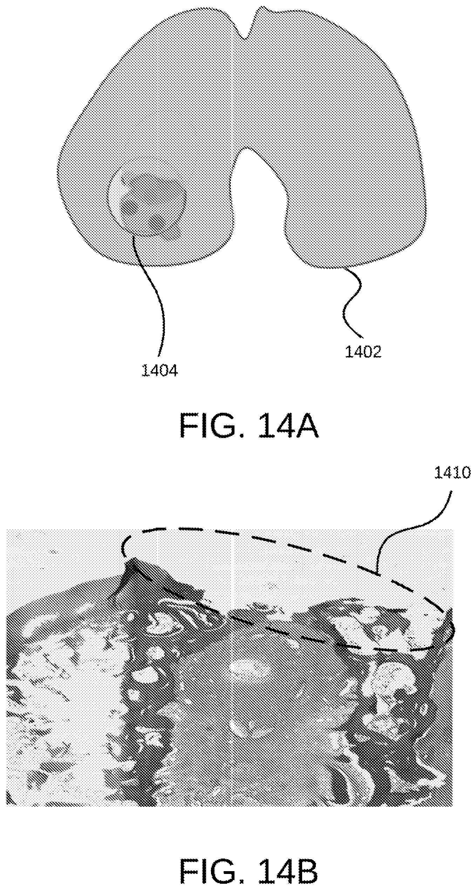

Second, the solid nTPU implants failed and became dislodged from the injury site. As a non-limiting, illustrative example, FIG. 14A shows femoral condyle cross-section 1403 associated with solid nTPU implant treatment area 1404 at the conclusion of the four-month period with implant dislodging and damage to existing tissue. Furthermore, as shown in FIG. 14B, which depicts an H&E histological stain of the solid nTPU implant treatment area after the four-month period of time, area 1410 shows bone damage.

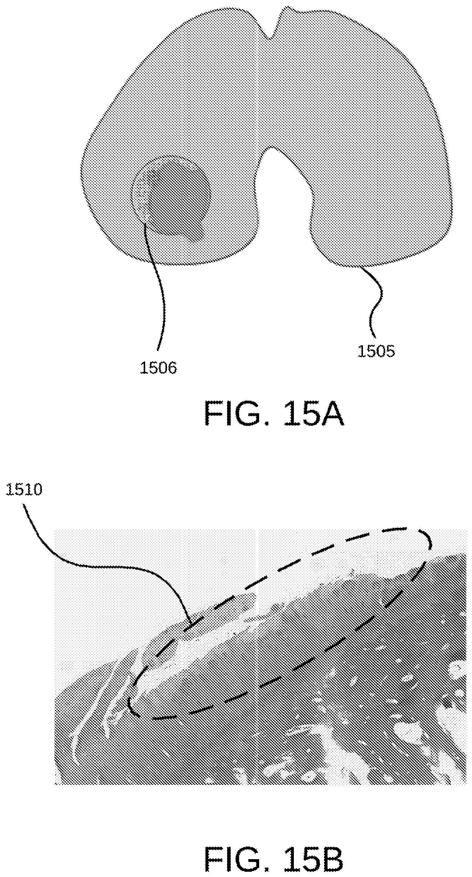

Third, the 3D printed nanoporous nTPU implants remained intact and in place with as much as 70% incorporation with new tissue. As a non-limiting, illustrative example, FIG. 15A shows femoral condyle cross-section 1505 associated with 3D printed nanoporous nTPU implant treatment area 1505 at the conclusion of the four-month period with as much as 70% incorporation with new tissue. Furthermore, as shown in FIG. 15B, which depicts an H&E histological stain of the 3D printed nanoporous nTPU implant treatment area after the four-month period of time, area 1510 shows formation of new cartilage tissue.

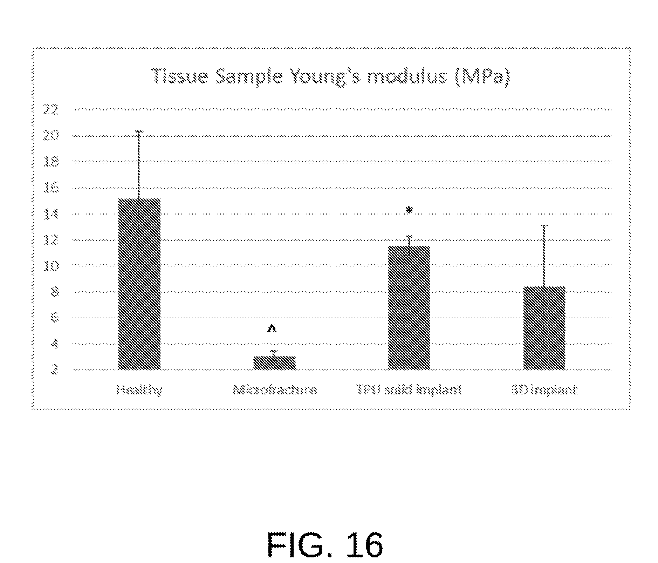

Further, mechanical testing showed that the 3D printed nanoporous nTPU implant treatment area had statistically equivalent mechanical properties when compared to healthy osteochondral tissue samples. For example, as shown in FIG 16, the 3D printed nanoporous nTPU implant treatment area exhibited a cartilage-like Young's modulus with no statistical difference between healthy cartilage tissue. Furthermore, the 3D printed nanoporous nTPU implant treatment area significantly outperformed the treatment area corresponding to the microfracture only treatment area in regard to exhibited Young's modulus.

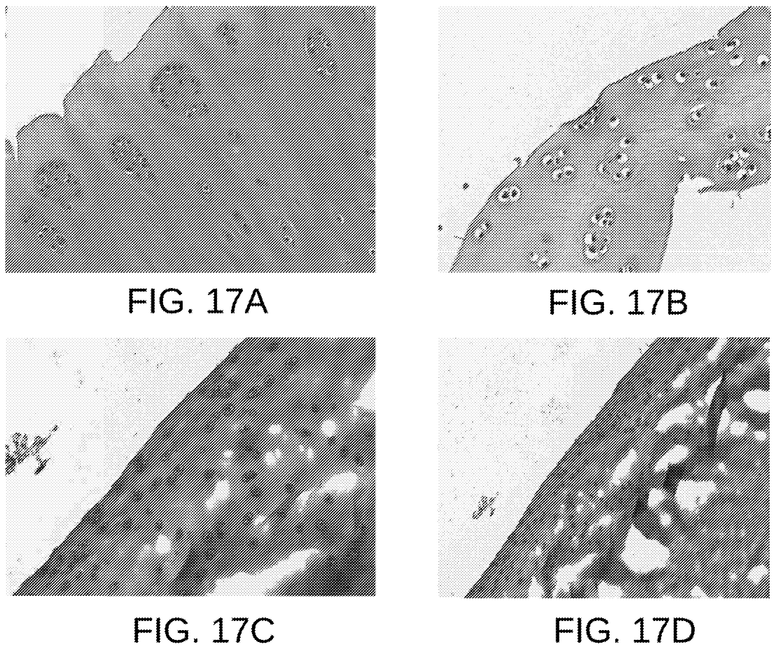

Finally, histological staining demonstrated that the 3D printed nanoporous nTPU implant displayed the most effective repair of the cartilage defect area. For example, while a thin layer of fibrocartilage was observed on the microfracture defect samples, the 3D printed nanoporous nTPU implant exhibited new cartilage formation, sometimes called "nuvo cartilage," as shown in FIGS. 17A, 17B, 17C, and 17D. Nuvo cartilage is characterized by an observable collagen matrix with tight clusters of chondrocytes embedded within. Other histological images showed chondrocytes partially invading and taking up residence in the nTPU material. This may be advantageous as it shows that the 3D printed nanoporous nTPU implant, as designed and validated, can support new healthy tissue growth, which may give a patient much longevity compared to the fibrocartilage that microfracture induces.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A three-dimensional tissue scaffold (100) comprising:
(a) a first region (112) comprising a plurality of layers (102), wherein at least a first layer of the plurality of layers is of a first rotational offset from at least a second layer of the plurality of layers;
(b) a second region (114) comprising a plurality of layers (104), wherein at least a first layer of the plurality of layers is of a second rotational offset from at least a second layer of the plurality of layers,
wherein the first rotational offset is greater than the second rotational offset; and
**characterized in that** the three-dimensional tissue scaffold further comprises
(c) a third region (116), positioned in between the first region and the second region, wherein the third region comprises a plurality of layers (106), and wherein at least a first layer of the plurality of layers of the third region is of a first sinusoidal pattern at a first rotation, at least a second layer of the plurality of layers of the third region is of the first sinusoidal pattern at the second rotation, and at least a third layer of the plurality of layers of the third region is of the first sinusoidal pattern at the third rotation

2. The three-dimensional tissue scaffold (100) of claim 1, wherein the plurality of layers (102) of the first region (112) further comprises a bottommost layer formed from at least a first boundary segment and at least a first crossing segment, and the plurality of layers (104) of the second region (114) further comprises a topmost layer formed from at least a second boundary segment and a second crossing segment, and wherein an exterior surface of at least the first boundary segment and at least the first crossing segment of the bottommost layer has a first topography comprising a plurality of peaks and valleys of a first average amplitude and an exterior surface of at least the second boundary segment and at least the second crossing segment of the topmost layer has a second topography comprising a plurality of peaks and valleys of a second average amplitude.

3. The three-dimensional tissue scaffold (100) of claim 2, wherein the first average amplitude is greater than the second average amplitude.

4. The three-dimensional tissue scaffold (100) of any one of claims 1-3, wherein at least the first layer of the plurality of layers (302) of the first region is of a first sinusoidal pattern at a first rotation and at least the second layer of the plurality of layers (302) of the first region is of the first sinusoidal pattern at a second rotation.

5. The three-dimensional tissue scaffold (100) of claim 4, wherein at least the first layer and second layer of the plurality of layers (302) of the first region are formed from a first number of boundary segments and a first number of crossing segments.

6. The three-dimensional tissue scaffold (100) of claim 5, wherein at least the first layer of the plurality of layers (306) of the second region is of a second sinusoidal pattern at the first rotation and at least the second layer of the plurality of layers (306) of the second region is of the second sinusoidal pattern at a third rotation.

7. The three-dimensional tissue scaffold (100) of claim 6, wherein at least the first layer and second layer of the plurality of layers (306) of the second region are formed from a second number of boundary segments and a second number of crossing segments.

8. The three-dimensional tissue scaffold (100) of claim 7, wherein the first number of boundary segments is less than the second number of boundary segments and the first number of crossing segments is less than the second number of boundary segments.

9. The three-dimensional tissue scaffold (100) of any one of claims 1-8, wherein each of the plurality of layers (304) of the third region comprises a number of boundary segments and a number of crossing segments, and wherein the number of boundary segments and the number of crossing segments increase on a layer-by-layer basis from a first number of boundary segments and a first number of crossing segments of the first layer of the plurality of layers (304) of the third region to a second number of boundary segments and a second number of crossing segments of the last layer of the plurality of layers (304) of the third region.

10. The three-dimensional tissue scaffold of claim 9, wherein the first number of boundary segments and the first number of crossing segments of the first layer of the plurality of layers (304) of the third region is equal to the first number of boundary segments and the first number of crossing segments of the first layer of the plurality of layers (302) of the first region.

11. The three-dimensional tissue scaffold of claim 9, wherein the second number of boundary segments and the second number of crossing segments of the last layer of the plurality of layers (304) of the third region is equal to the second number of boundary segments and the second number of crossing segments of the first layer of the plurality of layers of the second region.

12. The three-dimensional tissue scaffold of any one of claims 1-11, wherein the first sinusoidal pattern is a sinusoidal pattern around a constraining boundary of the third region (106).

13. The three-dimensional tissue scaffold of claim 12, wherein the constraining boundary of the third region (106) is a circle, an oval, a square, a regular geometric shape, or an irregular geometric shape.

## Patentansprüche

1. Dreidimensionales Gewebegerüst (100), umfassend:
(a) einen ersten Bereich (112), der eine Vielzahl von Schichten (102) umfasst, wobei mindestens eine erste Schicht der Vielzahl von Schichten einen ersten Rotationsversatz gegenüber mindestens einer zweiten Schicht der Vielzahl von Schichten aufweist;
(b) einen zweiten Bereich (114), der eine Vielzahl von Schichten (104) umfasst, wobei mindestens eine erste Schicht der Vielzahl von Schichten einen zweiten Rotationsversatz gegenüber mindestens einer zweiten Schicht der Vielzahl von Schichten aufweist,
wobei der erste Rotationsversatz größer als der zweite Rotationsversatz ist; und
**dadurch gekennzeichnet, dass** das dreidimensionale Gewebegerüst ferner Folgendes umfasst
(c) einen dritten Bereich (116), der zwischen dem ersten Bereich und dem zweiten Bereich positioniert ist, wobei der dritte Bereich eine Vielzahl von Schichten (106) umfasst, und wobei mindestens eine erste Schicht der Vielzahl von Schichten des dritten Bereichs aus einem ersten sinusförmigen Muster in einer ersten Rotation besteht, mindestens eine zweite Schicht der Vielzahl von Schichten des dritten Bereichs aus dem ersten sinusförmigen Muster in der zweiten Rotation besteht, und mindestens eine dritte Schicht der Vielzahl von Schichten des dritten Bereichs aus dem ersten sinusförmigen Muster in der dritten Rotation besteht.

2. Dreidimensionales Gewebegerüst (100) nach Anspruch 1, wobei die Vielzahl von Schichten (102) des ersten Bereichs (112) ferner eine unterste Schicht umfasst, die aus mindestens einem ersten Grenzsegment und mindestens einem ersten Kreuzungssegment gebildet ist, und die Vielzahl von Schichten (104) des zweiten Bereichs (114) ferner eine oberste Schicht umfasst, die aus mindestens einem zweiten Grenzsegment und einem zweiten Kreuzungssegment gebildet ist, und wobei eine Außenoberfläche von mindestens dem ersten Grenzsegment und mindestens dem ersten Kreuzungssegment der untersten Schicht eine erste Topographie aufweist, die eine Vielzahl von Spitzen und Tälern einer ersten durchschnittlichen Amplitude umfasst, und eine Außenoberfläche von mindestens dem zweiten Grenzsegment und mindestens dem zweiten Kreuzungssegment der obersten Schicht eine zweite Topographie aufweist, die eine Vielzahl von Spitzen und Tälern einer zweiten durchschnittlichen Amplitude umfasst.

3. Dreidimensionales Gewebegerüst (100) nach Anspruch 2, wobei die erste durchschnittliche Amplitude größer als die zweite durchschnittliche Amplitude ist.

4. Dreidimensionales Gewebegerüst (100) nach einem der Ansprüche 1-3, wobei mindestens die erste Schicht der Vielzahl von Schichten (302) des ersten Bereichs aus einem ersten sinusförmigen Muster in einer ersten Rotation besteht und mindestens die zweite Schicht der Vielzahl von Schichten (302) des ersten Bereichs aus dem ersten sinusförmigen Muster in einer zweiten Rotation besteht.

5. Dreidimensionales Gewebegerüst (100) nach Anspruch 4, wobei mindestens die erste Schicht und zweite Schicht der Vielzahl von Schichten (302) des ersten Bereichs aus einer ersten Anzahl von Grenzsegmenten und einer ersten Anzahl von Kreuzungssegmenten gebildet sind.

6. Dreidimensionales Gewebegerüst (100) nach Anspruch 5, wobei mindestens die erste Schicht der Vielzahl von Schichten (306) des zweiten Bereichs aus einem zweiten sinusförmigen Muster in der ersten Rotation besteht und mindestens die zweite Schicht der Vielzahl von Schichten (306) des zweiten Bereichs aus dem zweiten sinusförmigen Muster in einer dritten Rotation besteht.

7. Dreidimensionales Gewebegerüst (100) nach Anspruch 6, wobei mindestens die erste Schicht und zweite Schicht der Vielzahl von Schichten (306) des zweiten Bereichs aus einer zweiten Anzahl von Grenzsegmenten und einer zweiten Anzahl von Kreuzungssegmenten gebildet sind.

8. Dreidimensionales Gewebegerüst (100) nach Anspruch 7, wobei die erste Anzahl von Grenzsegmenten kleiner als die zweite Anzahl von Grenzsegmenten ist und die erste Anzahl von Kreuzungssegmenten kleiner als die zweite Anzahl von Grenzsegmenten ist.

9. Dreidimensionales Gewebegerüst (100) nach einem der Ansprüche 1-8, wobei jede der Vielzahl von Schichten (304) des dritten Bereichs eine Anzahl von Grenzsegmenten und eine Anzahl von Kreuzungssegmenten umfasst, und wobei die Anzahl von Grenzsegmenten und die Anzahl von Kreuzungssegmenten auf einer Schicht-für-Schicht-Basis von einer ersten Anzahl von Grenzsegmenten und einer ersten Anzahl von Kreuzungssegmenten der ersten Schicht der Vielzahl von Schichten (304) des dritten Bereichs auf eine zweite Anzahl von Grenzsegmenten und eine zweite Anzahl von Kreuzungssegmenten der letzten Schicht der Vielzahl von Schichten (304) des dritten Bereichs zunehmen.

10. Dreidimensionales Gewebegerüst nach Anspruch 9, wobei die erste Anzahl von Grenzsegmenten und die erste Anzahl von Kreuzungssegmenten der ersten Schicht der Vielzahl von Schichten (304) des dritten Bereichs gleich der ersten Anzahl von Grenzsegmenten und der ersten Anzahl von Kreuzungssegmenten der ersten Schicht der Vielzahl von Schichten (302) des ersten Bereichs ist.

11. Dreidimensionales Gewebegerüst nach Anspruch 9, wobei die zweite Anzahl von Grenzsegmenten und die zweite Anzahl von Kreuzungssegmenten der letzten Schicht der Vielzahl von Schichten (304) des dritten Bereichs gleich der zweiten Anzahl von Grenzsegmenten und der zweiten Anzahl von Kreuzungssegmenten der ersten Schicht der Vielzahl von Schichten des zweiten Bereichs ist.

12. Dreidimensionales Gewebegerüst nach einem der Ansprüche 1-11, wobei das erste sinusförmige Muster ein sinusförmiges Muster um eine begrenzende Grenze des dritten Bereichs (106) ist.

13. Dreidimensionales Gewebegerüst nach Anspruch 12, wobei die begrenzende Grenze des dritten Bereichs (106) ein Kreis, ein Oval, ein Quadrat, eine regelmäßige geometrische Form oder eine unregelmäßige geometrische Form ist.

## Revendications

1. Échafaudage tissulaire tridimensionnel (100) comprenant :
(a) une première région (112) comprenant une pluralité de couches (102), dans lequel au moins une première couche de la pluralité de couches présente un premier décalage rotationnel par rapport à au moins une deuxième couche de la pluralité de couches ;
(b) une deuxième région (114) comprenant une pluralité de couches (104), dans lequel au moins une première couche de la pluralité de couches présente un second décalage rotationnel par rapport à au moins une deuxième couche de la pluralité de couches,
dans lequel le premier décalage rotationnel est supérieur au second décalage rotationnel ; et
**caractérisé en ce que** l'échafaudage tissulaire tridimensionnel comprend en outre
(c) une troisième région (116), positionnée entre la première région et la deuxième région, dans lequel la troisième région comprend une pluralité de couches (106), et dans lequel au moins une première couche de la pluralité de couches de la troisième région présente un premier motif sinusoïdal lors d'une première rotation, au moins une deuxième couche de la pluralité de couches de la troisième région présente le premier motif sinusoïdal lors d'une deuxième rotation et au moins une troisième couche de la pluralité de couches de la troisième région présente le premier motif sinusoïdal lors d'une troisième rotation.

2. Échafaudage tissulaire tridimensionnel (100) selon la revendication 1, dans lequel la pluralité de couches (102) de la première région (112) comprend en outre une couche la plus basse formée à partir d'au moins un premier segment de bordure et d'au moins un premier segment de croisement, et la pluralité de couches (104) de la deuxième région (114) comprend en outre une couche la plus haute formée à partir d'au moins un second segment de bordure et d'un second segment de croisement, et dans lequel une surface extérieure d'au moins le premier segment de bordure et d'au moins le premier segment de croisement de la couche la plus basse a une première topographie comprenant une pluralité de crêtes et de vallées d'une première amplitude moyenne et une surface extérieure d'au moins le second segment de bordure et d'au moins le second segment de croisement de la couche la plus haute a une seconde topographie comprenant une pluralité de crêtes et de vallées d'une seconde amplitude moyenne.

3. Échafaudage tissulaire tridimensionnel (100) selon la revendication 2, dans lequel la première amplitude moyenne est supérieure à la seconde amplitude moyenne.

4. Échafaudage tissulaire tridimensionnel (100) selon l'une quelconque des revendications 1 à 3, dans lequel au moins la première couche de la pluralité de couches (302) de la première région présente un premier motif sinusoïdal lors d'une première rotation et au moins la deuxième couche de la pluralité de couches (302) de la première région présente le premier motif sinusoïdal lors d'une deuxième rotation.

5. Échafaudage tissulaire tridimensionnel (100) selon la revendication 4, dans lequel au moins la première couche et la deuxième couche de la pluralité de couches (302) de la première région sont formées à partir d'un premier nombre de segments de bordure et d'un premier nombre de segments de croisement.

6. Échafaudage tissulaire tridimensionnel (100) selon la revendication 5, dans lequel au moins la première couche de la pluralité de couches (306) de la deuxième région présente un second motif sinusoïdal lors de la première rotation et au moins la deuxième couche de la pluralité de couches (306) de la deuxième région présente le second motif sinusoïdal lors d'une troisième rotation.

7. Échafaudage tissulaire tridimensionnel (100) selon la revendication 6, dans lequel au moins la première couche et la deuxième couche de la pluralité de couches (306) de la deuxième région sont formées à partir d'un second nombre de segments de bordure et d'un second nombre de segments de croisement.

8. Échafaudage tissulaire tridimensionnel (100) selon la revendication 7, dans lequel le premier nombre de segments de bordure est inférieur au second nombre de segments de bordure et le premier nombre de segments de croisement est inférieur au second nombre de segments de bordure.

9. Échafaudage tissulaire tridimensionnel (100) selon l'une quelconque des revendications 1 à 8, dans lequel chacune de la pluralité de couches (304) de la troisième région comprend un nombre de segments de bordure et un nombre de segments de croisement, et dans lequel le nombre de segments de bordure et le nombre de segments de croisement augmentent couche par couche d'un premier nombre de segments de bordure et d'un premier nombre de segments de croisement de la première couche de la pluralité de couches (304) de la troisième région à un second nombre de segments de bordure et un second nombre de segments de croisement de la dernière couche de la pluralité de couches (304) de la troisième région.

10. Échafaudage tissulaire tridimensionnel selon la revendication 9, dans lequel le premier nombre de segments de bordure et le premier nombre de segments de croisement de la première couche de la pluralité de couches (304) de la troisième région sont égaux au premier nombre de segments de bordure et au premier nombre de segments de croisement de la première couche de la pluralité de couches (302) de la première région.

11. Échafaudage tissulaire tridimensionnel selon la revendication 9, dans lequel le second nombre de segments de bordure et le second nombre de segments de croisement de la dernière couche de la pluralité de couches (304) de la troisième région sont égaux au second nombre de segments de bordure et au second nombre de segments de croisement de la première couche de la pluralité de couches de la deuxième région.

12. Échafaudage tissulaire tridimensionnel selon l'une quelconque des revendications 1 à 11, dans lequel le premier motif sinusoïdal est un motif sinusoïdal autour d'une limite contraignante de la troisième région (106).

13. Échafaudage tissulaire tridimensionnel selon la revendication 12, dans lequel la limite contraignante de la troisième région (106) est un cercle, une ellipse, un carré, une forme géométrique régulière ou une forme géométrique irrégulière.
